# EUROPEAN PATENT APPLICATION

(11) **EP 2 684 864 A1**
(43) Date of publication of application: **15.01.2014**
(21) Application number: 12755499.6
(22) Date of filing: 22.02.2012
(51) Int. Cl.: C07C 227/34, C07C 227/10, C07C 227/16, C07C 229/48, C07B 57/00

(54) **METHOD OF PRODUCING OPTICALLY ACTIVE 1-AMINO-2-VINYLCYCLOPROPANE CARBOXYLIC ACID ESTER**

(30) Priority: 10.03.2011 JP 2011052639
(71) Applicant: Sumitomo Chemical Company Limited, Tokyo 104-8260 (JP)
(72) Inventor: AIKAWA, Toshiaki, Osaka-shi Osaka 555-0021 (JP)
(74) Representative: Nieuwenhuys, William Francis
(86) International application number: PCT/JP2012/055009
(87) International publication number: WO 2012/121068

(57) **Abstract**

An optically active 1-amino-2-vinylcyclopropanecarboxylic acid ester with high optical purity can be obtained by a method of producing an optically active 1-amino-2-vinylcyclopropanecarboxylic acid ester by reacting a 1-amino-2-vinylcyclopropanecarboxylic acid ester with an optically active tartaric acid or an optically active camphorsulfonic acid in a solvent, isolating one diastereomeric salt from the obtained diastereomeric salt mixture and treating the isolated diastereomeric salt with an inorganic acid or a base.

## Description

### TECHNICAL FIELD

The present invention relates to a method of producing an optically active 1-amino-2-vinylcyclopropanecarboxylic acid ester.

### BACKGROUND ART

Optically active 1-amino-2-vinylcyclopropanecarboxylic acid esters are useful, for example, for a synthetic intermediate of pharmaceuticals such as an antiviral agent. With regard to the method of producing the same, it is known to obtain methyl (1R, 2S)-1-amino-2-vinylcyclopropanecarboxylate having an optical purity of 97.2% e.e. (hereinafter, e.e. refers to enantiomeric excess) by optically resolving a racemic ethyl 1-amino-2-vinylcyclopropanecarboxylate by use of di-p-toluoyl-D-tartaric acid, converting the obtained ethyl (1R, 2S)-1-amino-2-vinylcyclopropanecarboxylate having an optical purity of 55% e.e. into a methyl ester by an ester exchange reaction and further optically resolving the resultant methyl ester by an enzyme reaction (Journal of Organic Chemistry, Volume 70, pages 5,869-5,879, 2005 (Supporting Information)).

In order to obtain an optically active 1-amino-2-vinylcyclopropanecarboxylic acid ester having a high optical purity, an enzyme that is high in substrate specificity is used in the above method. Accordingly, the method requires the conversion of the ethyl (1R, 2S)-1-amino-2-vinylcyclopropanecarboxylate having an optical purity of 55% e.e. into a methyl ester, and therefore, the method has a problem that the operation is complicated.

### SUMMARY OF THE INVENTION

The present invention provides a method capable of conveniently producing an optically active 1-amino-2-vinylcyclopropanecarboxylic acid ester having a high optical purity.

Specifically, the present invention provides a method of producing an optically active 1-amino-2-vinylcyclopropanecarboxylic acid ester by reacting a 1-amino-2vinylcyclopropanecarboxylic acid ester with optically active tartaric acid or optically active camphorsulfonic acid in a solvent to thereby obtain a mixture of diastereomeric salts and isolating one of the diastereomeric salts from the thus obtained mixture, and treating the isolated diastereomeric salt with an inorganic acid or a base.

### MODE FOR CARRYING OUT THE INVENTION

Hereinafter, the present invention will be described in detail. The method of producing an optically active 1-amino-2-vinylcyclopropanecarboxylic acid ester of the present invention includes a step of reacting a 1-amino-2-vinylcyclopropanecarboxylic acid ester with optically active tartaric acid or optically active camphorsulfonic acid (hereinafter may be referred to as an optically active organic acid) in a solvent to thereby obtain a mixture of diastereomeric salts and isolating one of the diastereomeric salts from the thus obtained mixture (first step) and a step of treating the isolated diastereomeric salt with an inorganic acid or a base (second step).

The 1-amino-2-vinylcyclopropanecarboxylic acid ester used in the production method of the present invention is generally a mixture of (1R, 2S)-isomer of a 1-amino-2-vinylcyclopropanecarboxylic acid ester with (1S, 2R) -isomer thereof. The mixture preferably includes one isomer in a larger amount than the other isomer. The optical purity of the mixture is, for example, 40% e.e. or more and less than 95% e.e., preferably 55% e.e. or more and less than 95% e.e., more preferably 70% e. e. or more and less than 90% e.e., and even more preferably 75% e.e. or more and less than 85% e.e.

The optically active organic acid used in the first step is optically active tartaric acid which is D-tartaric acid or L-tartaric acid; or optically active camphorsulfonic acid such as D-10-camphorsulfonic acid or L-10-camphorsulfonic acid.

The amount of the optically active organic acid used in the first step is generally 1 mol or more per 1 mol of the 1-amino-2- vinylcyclopropanecarboxylic acid ester, and from the view point of yield and economic efficiency, it is preferably 1 mol to 4 mol and more preferably 1 mol to 2 mol.

Examples of the solvent used for the reaction of the 1-amino-2-vinylcyclopropanecarboxylic acid ester with the optically active organic acid include aliphatic hydrocarbon solvents such as pentane, hexane, isohexane, heptane, isoheptane, octane, isooctane, nonane, isononane, decane, isodecane, undecane, dodecane, cyclopentane, cyclohexane, methylcyclohexane, t-butylcyclohexane, and petroleum ether; aromatic solvents such as benzene, toluene, ethylbenzene, isopropylbenzene, t-butylbenzene, xylene, mesitylene, monochlorobenzene, monofluorobenzene, α,α,α-trifluoromethylbenzene, 1,2-dichlorobenzene, 1,3-dichlorobenzene, 1,2,3-trichlorobenzene, and 1,2,4-trichlorobenzene; ether solvents such as tetrahydrofuran, methyltetrahydrofuran, 1,4-dioxane, diethyl ether, dipropyl ether, diisopropyl ether, dibutyl ether, dipentyl ether, dihexyl ether, diheptyl ether, dioctyl ether, t-butyl methyl ether, cyclopentyl methyl ether, 1,2-dimethoxyethane, diethylene glycol dimethyl ether, anisole, and diphenyl ether; alcohol solvents such as methanol, ethanol, 1-propanol, 2-propanol, 1-butanol, isobutyl alcohol, t-butyl alcohol, 1-pentanol, 2-pentanol, isopentyl alcohol, 1-hexanol, 2-hexanol, isohexyl alcohol, 1-heptanol, 2-heptanol, 3-heptanol, isoheptyl alcohol, ethylene glycol monomethyl ether, ethylene glycol monoethyl ether, ethylene glycol monopropyl ether, ethylene glycol monoisopropyl ether, ethylene glycol monobutyl ether, ethylene glycol monoisobutyl ether, ethylene glycol mono t-butyl ether, diethylene glycol monomethyl ether, diethylene glycol monoethyl ether, diethylene glycol monopropyl ether, diethylene glycol monoisopropyl ether, diethylene glycol monobutyl ether, diethylene glycol monoisobutyl ether, and diethylene glycol mono t-butyl ether; nitrile solvents such as acetonitrile, propionitrile, and benzonitrile; chlorinated aliphatic hydrocarbon solvents such as dichloromethane, chloroform, and 1,2-dichloroethane; ester solvents such as methyl acetate, ethyl acetate, propyl acetate, isopropyl acetate, butyl acetate, isobutyl acetate, t-butyl acetate, amyl acetate, isoamyl acetate, hexyl acetate, methyl propionate, ethyl propionate, propyl propionate, and isopropyl propionate; ketone solvents such as acetone, methyl ethyl ketone, methyl propyl ketone, methyl isopropyl ketone, methyl butyl ketone, methyl isobutyl ketone, diethyl ketone, cyclopentanone, and cyclohexanone; aprotic polar solvents such as dimethyl sulfoxide, sulfolane, N,N-dimethylformamide, N,N-dimethylacetamide, N,N-dimethylpropionamide, N-methylpyrrolidone, γ-butyrolactone, dimethyl carbonate, diethyl carbonate, ethylene carbonate, propylene carbonate, 1,3-dimethyl-2-imidazolidinone, and 1,3-dimethyl-3,4,5,6-tetrahydro-2(1H)-pyridinone; water; and mixtures thereof.

The solvent is preferably an aromatic solvent, a ketone solvent, an ester solvent, an alcohol solvent, an ether solvent or a mixture thereof; more preferably a mixed solvent of any one of an aromatic solvent, a ketone solvent, an ester solvent and an ether solvent with an alcohol solvent; even more preferably a mixture of toluene with an alcohol solvent; and particularly preferably a mixture of toluene with ethanol or a mixture of toluene with 2-propanol.

The amount of the solvent is preferably 1 to 50 mL and more preferably 3 to 30 mL per 1 g of the 1-amino-2-vinylcyclopropanecarboxylic acid ester, although it depends on the kind of solvent used.

The reaction of the 1-amino-2-vinylcyclopropanecarboxylic acid ester with the optically active organic acid can be conducted by, for example, mixing the solvent with the 1-amino-2-vinylcyclopropanecarboxylic acid ester and adding the optically active organic acid to the obtained mixture; or by mixing the solvent with the optically active organic acid and then adding the 1-amino-2-vinylcyclopropanecarboxylic acid ester to the obtained mixture.

The reaction temperature in the reaction of the 1-amino-2-vinylcyclopropanecarboxylic acid ester with the optically active organic acid is not particularly limited, but is preferably 0°C or more and not more than the boiling point of the solvent, and more preferably 0°C or more and not more than 40°C.

From the mixture of diastereomeric salts formed in the solvent, one diastereomeric salt which predominantly precipitates can be separated from the other diastereomeric salt by isolation. The precipitated diastereomeric salt is isolated by solid-liquid separation such as filtration or decantation. The obtained diastereomeric salt is a salt of the 1-amino-2-vinylcyclopropanecarboxylic acid ester and the optically active organic acid.

In the case where no precipitation of one diastereomeric salt from the mixture of diastereomeric salts in the solvent is observed, it is possible to predominantly precipitate one diastereomeric salt by adding one diastereomeric salt which is prepared in advance as a seed crystal to the mixture solution of the diastereomeric salts and then cooling the solution.

In the case where the precipitation of a diastereomeric salt is observed, the solution may be directly cooled. However, in order to improve the optical purity of the precipitated diastereomeric salt, it is preferred that the solution is heated to dissolve the precipitates and then cooled to thereby predominantly precipitate one diastereomeric salt. In such precipitation of diastereomeric salts, it is possible to use one diastereomeric salt which is prepared in advance as a seed crystal. A higher optical purity of the seed crystal is better, and the purity is preferably 90% e.e. or more, more preferably 95% e.e. or more, even more preferably 98% e.e. or more, and particularly preferably 99% e.e. or more.

When the mixture solution of the diastereomeric salts is heated, it is preferably heated up to a temperature or 30°C or more and not more than the boiling point of the solvent. In the cooling treatment, the solution is preferably cooled to a temperature of 0 to 25°C. In order to improve the optical purity of the precipitated diastereomeric salt, it is preferred that the solution is gradually cooled.

After isolation, the obtained one diastereomeric salt is preferably subjected to washing in order to improve the optical purity thereof. Used for the washing treatment is, for example, the same solvent as that used in the reaction of the 1-amino-2-vinylcyclopropanecarboxylic acid ester with the optically active organic acid. After the washing, it is preferable to perform drying. The drying can be carried out under the condition of ordinary pressure or a reduced pressure and preferably at a temperature selected within the range of 20 to 80°C.

The liquid resulted from the solid-liquid separation contains the other diastereomeric salt. It is also possible to obtain the other diastereomeric salt from the liquid phase by a usual method.

The diastereomeric salt may be subjected to purification to further improve its optical purity.

As the purification, recrystallization is preferred. The purification may be carried out by, for example, a method of dissolving a diastereomeric salt in a solvent and then cooled to precipitate a purified diastereomeric salt; a method of dissolving a diastereomeric salt in a solvent and then dropping a poor solvent in the obtained solution to thereby precipitate a purified diastereomeric salt; a method of dissolving a diastereomeric salt in a solvent and then removing the solvent by a distillation to thereby precipitate a purified diastereomeric salt; or a combination thereof. In the purification, it is also possible to add one diastereomeric salt prepared in advance as a seed crystal to the solution.

In the purification treatment, examples of the solvent that dissolves the diastereomeric salt include alcohol solvents such as methanol, ethanol, 1-propanol, 2-propanol, 1-butanol, isobutyl alcohol, t-butyl alcohol, 1-pentanol, 2-pentanol, isopentyl alcohol, 1-hexanol, 2-hexanol, isohexyl alcohol, 1-heptanol, 2-heptanol, 3-heptanol, isopeptyl alcohol, ethylene glycol monomethyl ether, ethylene glycol monoethyl ether, ethylene glycol monopropyl ether, ethylene glycol monoisopropyl ether, ethylene glycol monobutyl ether, ethylene glycol monoisobutyl ether, ethylene glycol mono t-butyl ether, diethylene glycol monomethyl ether, diethylene glycol monoethyl ether, diethylene glycol monopropyl ether, diethylene glycol monoisopropyl ether, diethylene glycol monobutyl ether, diethylene glycol monoisobutyl ether, and diethylene glycol mono t-butyl ether; water; and mixtures thereof. Alcohol solvents, water, and mixtures thereof are more preferred, and methanol, ethanol, and mixtures thereof are further preferred.

In the purification, it is possible to suitably control the amount of the solvent in which the diastereomeric salt is dissolved in accordance with the kind of the used solvent. A preferred amount used therein is 1 to 10 mL per 1 g of the diastereomeric salt. The diastereomeric salt is dissolved at a temperature of preferably 0 to 60°C, and more preferably 10 to 40°C.

Examples of the poor solvent used in the purification include aliphatic hydrocarbon solvents such as pentane, hexane, isohexane, heptane, isoheptane, octane, isooctane, nonane, isononane, decane, isodecane, undecane, dodecane, cyclopentane, cyclohexane, methylcyclohexane, t-butylcyclohexane and petroleum ether; aromatic solvents such as benzene, toluene, ethylbenzene, isopropylbenzene, t-butylbenzene, xylene, mesitylene, monochlorobenzene, monofluorobenzene, α,α,α-trifluoromethylbenzene, 1,2-dichlorobenzene, 1,3-dichlorobenzene, 1,2,3-trichlorobenzene and 1,2,4-trichlorobenzene; cyclic ether solvents such as tetrahydrofuran, methyltetrahydrofuran and 1,4-dioxane; ester solvents such as methyl acetate, ethyl acetate, propyl acetate, isopropyl acetate, butyl acetate, isobutyl acetate, t-butyl acetate, amyl acetate, isoamyl acetate, hexyl acetate, methyl propionate, ethyl propionate, propyl propionate and isopropyl propionate; and ketone solvents such as acetone, methyl ethyl ketone, methyl propyl ketone, methyl isopropyl ketone, methyl butyl ketone, methyl isobutyl ketone, diethyl ketone, cyclopentanone and cyclohexanone. The aromatic solvents are preferred and toluene is more preferred. The amount of the poor solvent used may be suitably controlled in accordance with the degree of precipitation of the purified diastereomeric salt.

When the diastereomeric salt is dissolved in a solvent and then cooled to precipitate a purified diastereomeric salt, the solution is preferably cooled to a temperature selected from the range of 0 to 25°C and preferably cooled by 3 to 10°C per hour.

The obtained diastereomeric salt is, for example, a salt of a (1R, 2S)-1-amino-2-vinylcyclopropanecarboxylic acid ester or a (1S, 2R)-1-amino-2 -vinylcyclopropanecarboxylic acid ester and an optically active organic acid. Specific examples thereof include a salt of ethyl (1R, 2S)-1-amino-2-vinylcyclopropanecarboxylate and L-tartaric acid; and a salt of ethyl (1R, 2S)-1-amino-2-vinylcyclopropanecarboxylate and D-10-camphorsulfonic acid.

The second step can be performed by mixing the isolated diastereomeric salt with an inorganic acid or a base. An optically active 1-amino-2-vinylcyclopropanecarboxylic acid ester can be obtained in this step.

The inorganic acid to be mixed with the diastereomeric salt usually has an acidity higher than that of the optically active organic acid. Specific examples thereof include hydrochloric acid, phosphoric acid and sulfuric acid. Preferred inorganic acids are hydrochloric acid and sulfuric acid. These inorganic acids may be used alone, or may be used as a mixture with the solvent described later.

The amount of the inorganic acid used is usually 1 mol or more of hydrochloric acid or usually 0.5 mol or more of sulfuric acid per 1 mol of the diastereomeric salt.

The diastereomeric salt is mixed with the inorganic salt preferably in a solvent. Examples of the solvent include aliphatic hydrocarbon solvents such as pentane, hexane, isohexane, heptane, isoheptane, octane, isooctane, nonane, isononane, decane, isodecane, undecane, dodecane, cyclopentane, cyclohexane, methylcyclohexane, t-butylcyclohexane, and petroleum ether; aromatic solvents such as benzene, toluene, ethylbenzene, isopropylbenzene, t-butylbenzene, xylene, mesitylene, monochlorobenzene, monofluorobenzene, α,α,α-trifluoromethylbenzene, 1,2-dichlorobenzene, 1,3-dichlorobenzene, 1,2,3-trichlorobenzene, and 1,2,4-trichlorobenzene; ether solvents such as tetrahydrofuran, methyltetrahydrofuran, 1,4-dioxane, diethyl ether, dipropyl ether, diisopropyl ether, dibutyl ether, dipentyl ether, dihexyl ether, diheptyl ether, dioctyl ether, t-butyl methyl ether, cyclopentyl methyl ether, 1,2-dimethoxyethane, diethylene glycol dimethyl ether, anisole, and diphenyl ether; alcohol solvents such as methanol, ethanol, 1-propanol, 2-propanol, 1-butanol, isobutyl alcohol, t-butylalcohol, 1-pentanol, 2-pentanol, isopentyl alcohol, 1-hexanol, 2-hexanol, isohexyl alcohol, 1-heptanol, 2-heptanol, 3-heptanol, isoheptyl alcohol, ethylene glycol monomethyl ether, ethylene glycol monoethyl ether, ethylene glycol monopropyl ether, ethylene glycol monoisopropyl ether, ethylene glycol monobutyl ether, ethylene glycol monoisobutyl. ether, ethylene glycol mono-t-butyl ether, diethylene glycol monomethyl ether, diethylene glycol monoethyl ether, diethylene glycol monopropyl ether, diethylene glycol monoisopropyl ether, diethylene glycol monobutyl ether, diethylene glycol monoisobutyl ether, and diethylene glycol mono-t-butyl ether; nitrile solvents such as acetonitrile, propionitrile, and benzonitrile; ester solvents such as ethyl acetate, propyl acetate, isopropyl acetate, butyl acetate, isobutyl acetate, t-butyl acetate, amyl acetate, and isoamyl acetate; ketone solvents such as acetone, methyl ethyl ketone, methyl isopropyl ketone, methyl isobutyl ketone, cyclopentanone, and cyclohexanone; chlorinated aliphatic hydrocarbon solvents such as dichloromethane, chloroform, and 1,2-dichloroethane; carboxylic acid solvents such as formic acid, acetic acid, and propionic acid; water; and mixtures thereof.

The solvent used in the mixing of the diastereomeric salt with the inorganic acid is preferably a mixed solvent of an aromatic solvent with a ketone solvent or an alcohol solvent, more preferably a mixed solvent of an aromatic solvent with an alcohol solvent. The amount of the solvent used is preferably 1 to 50 mL, and more preferably 3 to 30 mL per 1 g of the diastereomeric salt.

The mixing of the diastereomeric salt with the inorganic acid can be carried out by, for example, mixing the diastereomeric salt with the solvent and adding the inorganic acid thereto. The mixing is carried out at a temperature preferably in the range of 0 to 40°C and more preferably in the range of 0 to 30°C. The mixing time is not particularly limited, but is preferably in the range of 1 minute to 24 hours.

When an optically active 1-amino-2-vinylcyclopropanecarboxylic acid ester is precipitated as an acid addition salt in the mixture obtained by mixing the diastereomeric salt with the inorganic acid, it is possible to obtain the acid addition salt by subjecting the acid addition salt to solid-liquid separation such as filtration or decantation. When the acid addition salt is insufficiently precipitated or the acid addition salt is not precipitated, it is possible to obtain an optically active 1-amino-2-vinylcyclopropanecarboxylic acid ester as an acid addition salt by, for example, subjecting the obtained mixture to concentration, mixing with a solvent which hardly dissolves the salt, or cooling, to thereby precipitate an acid addition salt, and then subjecting the acid addition salt to solid-liquid separation such as filtration or decantation. The obtained acid addition salt may be purified by, for example, recrystallization. It is also possible to obtain an optically active 1-amino-2-vinylcyclopropanecarboxylic acid ester as a free base in the same manner as in the base treatment of a diastereomeric salt which will be described later.

Specific examples of the acid addition salts include addition salts of hydrochloric acid, phosphoric acid and sulfuric acid.

A filtrate obtained by the solid-liquid separation described above contains an optically active organic acid, which can be collected from the filtrate by a usual method and recycled in the present invention.

Examples of the base which is mixed with the diastereomeric salt include alkali metal hydroxides such as potassium hydroxide and sodium hydroxide; alkali metal carbonates such as sodium carbonate and potassium carbonate; and alkali metal alcoholates such as sodium methylate, sodium ethylate, potassium methylate and potassium ethylate. Preferred bases are alkali metal hydroxides, and particularly preferred is sodium hydroxide. The bases may be used alone, or may be used as a mixture with the solvent described later.

The amount of the base used is preferably 1 mol or more per 1 mol of the diastereomeric salt.

The mixing of the diastereomeric salt with the base is preferably carried out in a solvent. Examples of such a solvent include alcohol solvents such as methanol, ethanol, 2-propanol, 1-propanol and 1-butanol; ether solvents such as diethyl ether, t-butylmethyl ether, methyl isobutyl ether, diisopropyl ether, methyl cyclopentyl ether and 1,2-dimethoxymethane; aromatic solvents such as toluene, xylene and chlorobenzene; aliphatic hydrocarbon solvents such as hexane and cyclohexane; ketone solvents such as methyl ethyl ketone and methyl isobutyl ketone; ester solvents such as ethyl acetate and t-butyl acetate; halogenated aliphatic hydrocarbon solvents such as dichloromethane; water; and mixtures thereof. Preferred solvents are aromatic solvents, alcohol solvents, water and mixtures thereof, and more preferred are toluene, water and a mixture thereof. When using a base such as an alkali metal hydroxide or an alkali metal carbonate as the base, the solvent is preferably water alone or a mixture of water with an organic solvent which has low compatibility with water (for example, the above-described ether solvents, aromatic solvents, aliphatic hydrocarbon solvents, ketone solvents, ester solvents or halogenated aliphatic hydrocarbon solvents).

The amount of the solvent used in the mixing of the diastereomeric salt with the base is preferably 1 to 50 mL, and more preferably 3 to 30 mL per 1 g of the diastereomeric salt.

The mixing of the diastereomeric salt with the base can be carried out by, for example, mixing the diastereomeric salt with the solvent and adding the base thereto. The mixing is carried out at a temperature preferably in the range of 0 to 60°C, and more preferably in the range of 10 to 30°C. The mixing time is not particularly limited, but is preferably in the range of 1 minute to 24 hours.

The mixing of the diastereomeric salt with the base can be carried out by, for example, the following process.

A base is added to mixture of water and the diastereomeric salt to make the aqueous layer of the mixture basic (preferably to a pH of 8.5 or more). An organic solvent having low compatibility with water is added to the obtained mixture and the mixture is subjected to liquid separation to obtain an organic layer containing an optically active 1-amino-2-vinylcyclopropanecarboxylic acid ester. The thus obtained organic layer is washed with water as needed, and then concentrated, and in this manner, it is possible to obtain an optically active 1-amino-2-vinylcyclopropanecarboxylic acid ester as a free base. When an alkali metal alcoholate is used as the base, and an alcohol solvent is used as the solvent, it is possible to precipitate an alkali metal salt of an optically active organic acid. By filtering off the precipitate and concentrating the resultant filtrate, it is possible to isolate an optically active 1-amino-2-vinylcyclopropanecarboxylic acid ester as a free base. The obtained optically active 1-amino-2-vinylcyclopropanecarboxylic acid ester may be purified by means of, for example, column chromatography.

An aqueous layer obtained by the liquid separation described above contains an optically active organic acid which can be recovered from the aqueous layer by a usual method and recycled in the present invention. Further, from the alkali metal salt of the optically active organic acid which has been filtered out in the process above, it is possible to recover an optically active organic acid by a usual method and recycle it in the present invention.

It is also possible to further mix an acid to the optically active 1-amino-2-vinylcyclopropanecarboxylic acid ester obtained by mixing a diastereomeric salt with a base to thereby obtain an acid addition salt of the optically active 1-amino-2-vinylcyclopropanecarboxylic acid ester.

Examples of the acid which is mixed with the optically active 1-amino-2-vinylcyclopropanecarboxylic acid ester include inorganic acids such as hydrochloric acid, sulfuric acid, phosphoric acid, nitric acid and perchloric acid; aromatic sulfonic acids such as p-toluenesulfonic acid and benzenesulfonic acid; aliphatic sulfonic acids such as methanesulfonic acid; aliphatic carboxylic acids such as acetic acid, propionic acid, citric acid, malic acid, succinic acid, lactic acid, maleic acid and fumaric acid; and aromatic carboxylic acids such as phthalic acid, benzoic acid, 4-nitrobenzoic acid and 4-chlorobenzoic acid.

The acids may be used alone, or may be used as a mixture with the solvent described later. The acid is preferably an inorganic acid and more preferably sulfuric acid.

The amount of the acid used is preferably, for example, 1 mol or more of hydrochloric acid or 0.5 mol or more of sulfuric acid per 1 mol of the optically active 1-amino-2-vinylcyclopropane carboxylic acid ester.

The optically active 1-amino-2-vinylcyclopropanecarboxylic acid ester is mixed with the acid preferably in a solvent. Examples of the solvent include aliphatic hydrocarbon solvents such as pentane, hexane, isohexane, heptane, isoheptane, octane, isooctane, nonane, isononane, decane, isodecane, undecane, dodecane, cyclopentane, cyclohexane, methylcyclohexane, t-butylcyclohexane, and petroleum ether; aromatic solvents such as benzene, toluene, ethylbenzene, isopropylbenzene, t-butylbenzene, xylene, mesitylene, monochlorobenzene, monofluorobenzene, α,α,α-trifluoromethylbenzene, 1,2-dichlorobenzene, 1,3-dichlorobenzene, 1,2,3-trichlorobenzene, and 1,2,4-trichlorobenzene; ether solvents such as tetrahydrofuran, methyltetrahydrofuran, 1,4-dioxane, diethyl ether, dipropyl ether, diisopropyl ether, dibutyl ether, dipentyl ether, dihexyl ether, diheptyl ether, dioctyl ether, t-butyl methyl ether, cyclopentyl methyl ether, 1,2-dimethoxyethane, diethylene glycol dimethyl ether, anisole, and diphenyl ether; alcohol solvents such as methanol, ethanol, 1-propanol, 2-propanol, 1-butanol, isobutyl alcohol, t-butyl alcohol, 1-pentanol, 2-pentanol, isopentyl alcohol, 1-hexanol, 2-hexanol, isohexyl alcohol, 1-heptanol, 2-heptanol, 3-heptanol, isopeptyl alcohol, ethylene glycol monomethyl ether, ethylene glycol monoethyl ether, ethylene glycol monopropyl ether, ethylene glycol monoisopropyl ether, ethylene glycol monobutyl ether, ethylene glycol monoisobutyl ether, ethylene glycol mono-t-butyl ether, diethylene glycol monomethyl ether, diethylene glycol monoethyl ether, diethylene glycol monopropyl ether, diethylene glycol monoisopropyl ether, diethylene glycol monobutyl ether, diethylene glycol monoisobutyl ether, and diethylene glycol mono-t-butyl ether; nitrile solvents such as acetonitrile, propionitrile, and benzonitrile; ester solvents such as ethyl acetate, propyl acetate, isopropyl acetate, butyl acetate, isobutyl acetate, t-butyl acetate, amyl acetate, and isoamyl acetate; ketone solvents such as acetone, methyl ethyl ketone, methyl isopropyl ketone, methyl isobutyl ketone, cyclopentanone, and cyclohexanone; chlorinated aliphatic hydrocarbon solvents such as dichloromethane, chloroform, and 1,2-dichloroethane; water; and mixtures thereof.

The solvent which is used in the mixing of the optically active 1-amino-2-vinylcyclopropanecarboxylic acid ester with the acid is preferably a mixture of an aromatic solvent with a ketone solvent or an alcohol solvent; and more preferably a mixture of an aromatic solvent with an alcohol solvent. The amount of the solvent used per 1 g of the optically active 1-amino-2-vinylcyclopropanecarboxylic acid ester is preferably 1 to 50 mL, and more preferably 3 to 30 mL.

The mixing of the optically active 1-amino-2-vinylcyclopropanecarboxylic acid ester with the acid can be performed by, for example, mixing the optically active 1-amino-2-vinylcyclopropanecarboxylic acid ester with a solvent and adding the acid thereto. The mixing is carried out at a temperature preferably in the range of 0 to 40°C, and more preferably in the range of 0 to 30°C. The mixing time is not particularly limited, but is preferably in the range of 1 minute to 24 hours.

When an acid addition salt of the optically active 1-amino-2-vinylcyclopropanecarboxylic acid ester is precipitated in the mixture obtained by mixing the optically active 1-amino-2-vinylcyclopropanecarboxylic acid ester with the acid, it is possible to obtain the acid addition salt by, for example, subjecting the acid addition salt to solid-liquid separation such as filtration or decantation. When the acid addition salt is insufficiently precipitated or the acid addition salt is not precipitated, it is possible to collect the acid addition salt by, for example, subj ecting the obtained mixture to concentration, mixing with a solvent which hardly dissolve the salt, or cooling, to thereby precipitate the acid addition salt and then subjecting the acid addition salt to solid-liquid separation such as filtration or decantation. The collected acid addition salt may be purified by, for example, recrystallization.

Specific examples of the acid addition salt include addition salts of hydrochloric acid, phosphoric acid and sulfuric acid.

The optical purity of the obtained optically active 1-amino-2-vinylcyclopropanecarboxylic acid ester is, for example, 85% e.e. or more, for example, 90% e.e. or more, and for example, 98% e.e. or more.

Specific examples of the obtained optically active 1-amino-2-vinylcyclopropanecarboxylic acid ester include ethyl (1S,2R)-1-amino-2-vinylcyclopropanecarboxylate, t-butyl (1S,2R)-1-amino-2-vinylcyclopropanecarboxylate, t-butyl (1S,2R)-1-amino-2-vinylcyclopropanecarboxylate, methyl (1S,2R)-1-amino-2-vinylcyclopropanecarboxylate, ethyl (1R,2S)-1-amino-2-vinylcycloproapanecarboxylate, t-butyl (1R,2S)-1-amino-2-trinylcyclopropanecarboxylate, methyl (1R,2S)-1-amino-2-vinylcyclopropanecarboxylate, and enantiomers thereof.

The 1-amino-2-vinylcyclopropanecarboxylic acid ester used in the production method of the present invention can be prepared by a known method. For example, it can be prepared by the method in Journal of Organic Chemistry, volume 70, pages 5,869-5,879, 2005, wherein N-phenylmethyleneglycine ethyl ester is allowed to react with 1,4-dibromo-2-butene in the presence of a base, and the obtained ethyl 1-(N-phenylmethyleneamino)-2-vinylcyclopropanecarboxylate is subjected to an acid treatment or the like, and then the obtained racemic ethyl 1-amino-2-vinylcyclopropanecarboxylate is subjected to optical resolution or the like by use of di-p-toluoyl-D-tartaric acid. When a salt is formed from the 1-amino-2-vinylcyclopropanecarboxylic acid ester with an acid other than an optically active organic acid, it is preferable that the salt is subjected to a base treatment before being reacted with the optically active organic acid.

The 1-amino-2-vinylcyclopropanecarboxylic acid ester used in the production method of the present invention is preferably a compound (compound (4-2)) represented by Formula (4-2): 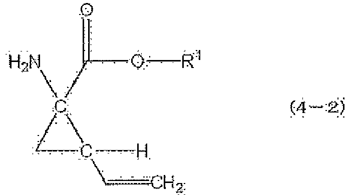 (wherein R¹ represents an alkyl group having 1 to 12 carbon atoms or an alkenyl group having 2 to 12 carbon atoms). Compound (4-2) is preferably prepared by reacting a compound (compound (1)) represented by Formula (1): 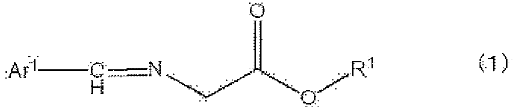 (wherein R¹ represents the same as the above and Ar¹ represents an optionally substituted phenyl group or an optionally substituted naphthyl group) with a compound (compound (2)) represented by Formula (2): 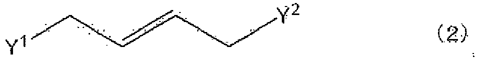 (wherein Y¹ and Y² represent each independently a halogen atom, an alkanesulfonyloxy group having 1 to 6 carbon atoms, a perfluoroalkanesulfonyloxy group having 1 to 6 carbon atoms, or an optionally substituted benzenesulfonyloxy group. The substituents for the benzenesulfonyloxy group are one or more substituents selected from an alkyl group having 1 to 6 carbon atoms, a halogen atom and a nitro group) in the presence of an optically active quaternary ammonium salt, to thereby obtain a compound (compound (3)) represented by Formula (3): 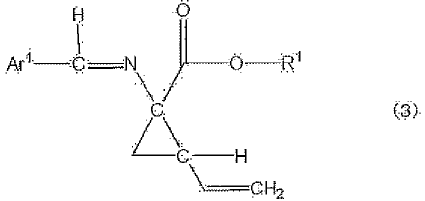 (wherein Ar¹ and R¹ represent the same as the above) and subjecting the thus obtained compound (3) to imine hydrolysis.

In that case, the obtained optically active 1-amino-2-vinylcyclopropanecarboxylic acid ester is a compound represented by Formula (4): 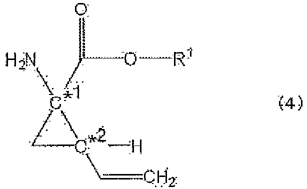 (wherein R¹ represents the same as the above, C*¹ and C*² each represent an asymmetric carbon atom, C*² has an S configuration when C*¹ has an R configuration, and C*² has an R configuration when C*¹ has an S configuration).

Examples of the alkyl group having 1 to 12 carbon atoms represented by R¹ include straight-chain or branched alkyl groups having 1 to 12 carbon atoms such as a methyl group, an ethyl group, a propyl group, an isopropyl group, a butyl group, an isobutyl group, a t-butyl group, a pentyl group, a hexyl group, a heptyl group, an octyl group, a nonyl group, a decyl group, an undecyl group and a dodecyl group; and cyclic alkyl groups having 3 to 12 carbon atoms such as a cyclopropyl group, a cyclobutyl group, a cyclopentyl group, a cyclohexyl group, a cycloheptyl group and a cyclooctyl group. Examples of the alkenyl group having 2 to 12 carbon atoms represented by R¹ include straight-chain or branched alkenyl groups such as an ethenyl group, a 2-propenyl group, a 2-butenyl group and a 3-methyl-2-butenyl group; and cyclic alkenyl groups such as a 1-cyclohexenyl group.

R¹ is preferably an alkyl group having 1 to 12 carbon atoms, more preferably a methyl group, an ethyl group or a t-butyl group, and even more preferably a methyl group or an ethyl group.

In Formula (1) and Formula (3), the phenyl group or the naphthyl group represented by Ar¹ may be substituted. Examples of a substituent therefor include at least one group selected from Group P1 below.

### <Group P1>

Alkyl groups having 1 to 12 carbon atoms, alkoxy groups having 1 to 12 carbon atoms, a halogen atom, a nitro group, a cyano group and a trifluoromethyl group.

In Group P1, examples of the alkyl groups having 1 to 12 carbon atoms include straight-chain or branched alkyl groups having 1 to 12 carbon atoms such as a methyl group, an ethyl group, a propyl group, an isopropyl group, a butyl group, an isobutyl group, a t-butyl group, a pentyl group, a hexyl group, a heptyl group, an octyl group, a nonyl group, a decyl group, an undecyl group and a dodecyl group; and cyclic alkyl groups having 3 to 12 carbon atoms such as a cyclopropyl group, a cyclobutyl group, a cyclopentyl group, a cyclohexyl group, a cycloheptyl group and a cyclooctyl group. Examples of the alkoxy group having 1 to 12 carbon atoms include straight-chain or branched alkoxy groups having 1 to 12 carbon atoms such as a methoxy group, an ethoxy group, a propyloxy group, an isopropyloxy group, a butyloxy group, an isobutyloxy group, a t-butyloxy group, a pentyloxy group, a hexyloxy group, a heptyloxy group and an octyloxy group; and cyclic alkyloxy groups having 3 to 12 carbon atoms such as a cyclopropyloxy group, a cyclobutyloxy group, a cyclopentyloxy group, a cyclohexyloxy group, a cycloheptyloxy group and a cyclooctyloxy group. Examples of the halogen atom include a fluorine atom, a chlorine atom and a bromine atom.

Examples of the optionally substituted phenyl group represented by Ar¹ and the optionally substituted naphthyl group represented by Ar¹ include a phenyl group, a 1-naphthyl group, a 2-naphthyl group, a 2-methylphenyl group, a 2-methoxyphenyl group, a 2-fluorophenyl group, a 2-chlorophenyl group, a 2-bromophenyl group, a 2-nitrophenyl group, a 2-cyanophenyl group, a 2-(trifluoromethyl)phenyl group, a 3-methylphenyl group, a 3-methoxyphenyl group, a 3-fluorophenyl group, a 3-chlorophenyl group, a 3-bromophenyl group, a 3-nitrophenyl group, a 3-cyanophenyl group, a 3-(trifluoromethyl)phenyl group, a 4-methylphenyl group, a 4-methoxyphenyl group, a 4-fluorophenyl group, a 4-chlorophenyl group, a 4-bromophenyl group, a 4-nitrophenyl group, a 4-cyanophenyl group, a 4-(trifluoromethyl)phenyl group, a 2,3-dichlorophenyl group, a 2,4-dichlorophenyl group, a 3,4-dichlorophenyl group, and a 3,4,5-trichlorophenyl group.

Ar¹ is preferably an optionally substituted phenyl group, more preferably a phenyl group optionally substituted by halogen and even more preferably a phenyl group or a 4-chlorophenyl group.

As for Y¹ and Y² in Formula (2), examples of the halogen atom include a chlorine atom, a bromine atom and an iodine atom; examples of the alkanesulfonyloxy group having 1 to 6 carbon atoms include a methanesulfonyloxy group, an ethanesulfonyloxy group, a propanesulfonyloxy group, a butanesulfonyloxy group, a pentanesulfonyloxy group and a hexanesulfonyloxy group; and examples of the perfluoroalkanesulfonyloxy group having 1 to 6 carbon atoms include a trifluoromethanesulfonyloxy group, a pentafluoroethanesulfonyloxy group, a perfluoropropanesulfonyloxy group and a perfluorohexanesulfonyloxy group.

As for Y¹ or Y² in Formula (2), the hydrogen atom on the benzenesulfonyloxy group may be each independently substituted by, for example, a group selected from the group consisting of an alkyl group having 1 to 6 carbon atoms, a halogen atom and a nitro group. Examples of the alkyl group having 1 to 6 carbon atoms include a methyl group, an ethyl group, a propyl group, an isopropyl group, a butyl group and a t-butyl group. Examples of the halogen atom include a fluorine atom, a chlorine atom and a bromine atom. Examples of the optionally substituted benzenesulfonyloxy group include a 4-methylbenzenesulfonyloxy group, a 2-nitrobenzenesulfonyloxy group, a 3-nitrobenzenesulfonyloxy group, a 4-nitrobenzenesulfonyloxy group, a 2,4-di-nitrobenzenesulfonyloxy group, a 4-fluorobenzenesulfonyloxy group and a pentafluorobenzenesulfonyloxy group.

Y¹ and Y² are preferably each independently a chlorine atom, a bromine atom or a methanesulfonyloxy group, and more preferably, Y¹ and Y² are both bromine atoms.

Specific examples of compound (1) include
N-phenylmethyleneglycine ethyl ester,
N-naphthalen-1-ylmethyleneglycine ethyl ester,
N-naphthalene-2-ylmethyleneglycine ethyl ester,
N-furan-2-ylmethyleneglycine ethyl ester,
N-(4-methylphenyl)methyleneglycine ethyl ester,
N-(4-methoxyphenyl)methyleneglycine ethyl ester,
N-(4-fluorophenyl)methyleneglycine ethyl ester,
N-(4-chlorophenyl)methyleneglycine ethyl ester,
N-[4-(trifluoromethyl)phenyl]methyleneglycine ethyl ester,
N-(3-chlorophenyl)methyleneglycine ethyl ester,
N-(4-chlorophenyl)methyleneglycine ethyl ester,
N-phenylmethyleneglycine t-butyl ester,
N-(4-chlorophenyl)methyleneglycine t-butyl ester,
N-phenylmethyleneglycine methyl ester and
N-(4-chlorophenyl)methyleneglycine methyl ester.

Compound (1) is preferably N-phenylmethyleneglycine ethyl ester, N-naphthalen-1-ylmethyleneglycine ethyl ester or N-(4-chlorophenyl)methyleneglycine ethyl ester.

Compound (1) can be prepared by any of known methods. It is also possible to use a product of compound (1) on the market.

Specific examples of compound (2) include
(E)-1,4-dibromo-2-butene, (E)-1,4-dichloro-2-butene,
(E)-1,4-dimethanesulfonyloxy-2-butene and
(E)-1-bromo-4-chloro-2-butene. Compound (2) is preferably
(E)-1,4-dibromo-2-butene or (E)-1,4-dichloro-2-butene, and more preferably (E)-1,4-dibromo-2-butene.

Compound (2) can be prepared according to any of known methods. It is also possible to use a product of compound (2) on the market.

Specific examples of compound (3) include ethyl (1S, 2R) - 1-(N-phenylmethylene)amino-2-vinylcyclopropanecarboxylate, ethyl (1S,2R)-1-[N-(4-chlorophenyl)methylene]amino-2-vinylcyclopropanecarboxylate, t-butyl (1S, 2R)-1-(N-phenylmethylene)amino-2-vinylcyclopropanecarboxylate, t-butyl (1S,2R)-1-[N-(4-chlorophenyl)methylene]amino-2-vinylcyclopropanecarboxylate, methyl (1S,2R)-1-(N-phenylmethylene)amino-2-vinylcyclopropanecarboxylate, methyl (1S,2R)-1-[N-(4-chlorophenyl)methylene]amino-2-vinylcyclopropanecarboxylate, ethyl (1S,2R)-1-(N-naphtalene-1-ylmethylene)amino-2-vinylcyclopropanecarboxylate, ethyl (1R,2S)-1-(N-phenylmethylene)amino-2-vinylcyclopropanecarboxylate, ethyl (1R,2S)-1-[N-(4-chlorophenyl)methylene]amino-2-vinylcyclopropanecarboxylate, t-butyl (1R,2S)-1-(N-phenylmethylene)amino-2-vinylcyclopropanecarboxylate, t-butyl (1R,2S)-1-[N-(4-chlorophenyl)methylene]amino-2-vinylcyclopropanecarboxylate, methyl (1R,2S)-1-(N-phenylmethylene)amino-2-vinylcyclopropanecarboxylate, methyl (1R,2S)-1-[N-(4-chlorophenyl)methylene]amino-2-vinylcyclopropanecarboxylate, ethyl (1R,2S)-1-(N-naphthalen-1-ylmethylene)amino-2-vinylcyclopropanecarboxylate, and optically active mixtures thereof with their enantiomers.

Examples of the optically active quaternary ammonium salts used in the reaction of compound (1) with compound (2) include a cinchona alkaloid derivative (see e.g., Tetrahedron Letters, Volume 40, pages 8,671-8,674, 1999); a tartaric acid derivative (see e.g., Tetrahedron, Volume 60, pages 7,743-7,754, 2004) and an axially asymmetric spiro quaternary ammonium salt (see e.g., Joural of American Chemical Society, Volume 122, pages 5,228-5,229, 2000). Preferred quaternary ammonium salts include a compound (compound (5)) represented by Formula (5): 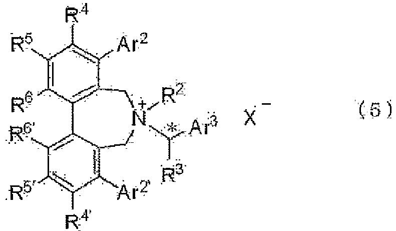 (wherein Ar² and Ar^{2'} represent each independently an optionally substituted phenyl group; Ar³ represents an optionally substituted aromatic hydrocarbon group having 6 to 20 carbon atoms or an optionally substituted aliphatic hydrocarbon group having 1 to 20 carbon atoms; R² represents an optionally substituted aliphatic hydrocarbon group having 1 to 12 carbon atoms and R³ represents a straight-chain hydrocarbon group having 1 to 12 carbon atoms, or alternatively, R² and R³ in combination form a polymethylene group having 2 to 6 carbon atoms; R⁴, R^{4'}, R⁵, R^{5'}, R⁶ and R^{6'} represent each independently a hydrogen atom, an aliphatic hydrocarbon group having 1 to 12 carbon atoms or an alkoxy group having 1 to 12 carbon atoms; * represents an asymmetric carbon atom; and X⁻ represents a monovalent anion).

The phenyl groups represented by Ar² and Ar^{2'} in Formula (5) may be substituted. Examples of the substituents therefor include the same groups as those selected from Group P1 listed above.

Examples of the optionally substituted phenyl groups represented by Ar² and Ar^{2'} include a phenyl group, a 2-methylphenyl group, a 3-methylphenyl group, a 4-methylphenyl group, a 3,5-dimethylphenyl group, a 3,4,5-trimethylphenyl group, a 2-t-butylphenyl group, a 3-t-butylphenyl group, a 4-t-butylphenyl group, a 2-t-butyloxyphenyl group, a 3-t-butyloxyphenyl group, a 4-t-butyloxyphenyl group, a 2-fluorophenyl group, a 3-fluorophenyl group, a 4-fluorophenyl group, a 3,5-difluorophenyl group, a 3,4,5-trifluorophenyl group, a 2-chlorophenyl group, a 3-chlorophenyl group, a 4-chlorophenyl group, a 3,5-dichlorophenyl group, a 3,4,5-trichlorophenyl group, a 2-(trifluoromethyl)phenyl group, a 3-(trifluoromethyl)phenyl group, a 4-(trifluoromethyl)phenyl group, a 3,5-bis(trifluoromethyl)phenyl group and a 3,5-difluoro-4-(trifluoromethyl)phenyl group.

Ar² and Ar^{2'} are preferably each independently a 3-fluorophenyl group, a 4-fluorophenyl group, a 3,5-difluorophenyl group, a 3,4,5-trifluorophenyl group or a 3,5-bis(trifluoromethyl)phenyl group; more preferably each independently a 3,4,5-trifluorophenyl group or a 3,5-bis(trifluoromethyl)phenyl group; and even more preferably both of Ar² and Ar^{2'} are 3,5-bis(trifluoromethyl)phenyl groups.

Examples of the optionally substituted aromatic hydrocarbon group having 6 to 20 carbon atoms represented by Ar³ include a phenyl group, a 1-naphthyl group, a 2-naphthyl group, a benzyl group, a 2-tolyl group, a 1,5-diphenyl-3-pentyl group, a bis(4-tolyl)methyl group, a 1,3-diphenyl-2-propyl group and a bis(3,4-dimethylphenyl)methyl group. Examples of the optionally substituted aliphatic hydrocarbon group having 1 to 20 carbon atoms represented by Ar³ include straight-chain alkyl groups having 1 to 20 carbon atoms such as a methyl group, an ethyl group, a propyl group, a butyl group, a pentyl group, a hexyl group, a heptyl group, an octyl group, a nonyl group, a decyl group, an undecyl group and a dodecyl group; branched alkyl groups having 3 to 20 carbon atoms such as a 1-methylethyl group, a 1-methylpropyl group, a 1-ethylpropyl group, a 1-propylbutyl group, a 1-butylpentyl group, a 1-pentylhexyl group, a 1-hexylheptyl group, a 1-heptyloctyl group, a 1-octylnonyl group and a 1-nonylundecyl group; cyclic alkyl groups having 3 to 20 carbon atoms such as a cyclopropyl group, a cyclobutyl group, a cyclopentyl group, a cyclohexyl group, a cycloheptyl group and a cyclooctyl group; straight-chain alkenyl groups having 2 to 20 carbon atoms such as an ethenyl group, a 1-propenyl group, a 2-propenyl group, a 1-butenyl group, a 1-pentenyl group, a 1-hexenyl group, a 1-heptenyl group, a 1-octenyl group and a 1-undecenyl group; and straight-chain alkynyl groups having 2 to 20 carbon atoms such as an ethynyl group, a 1-propynyl group, a 2-propynyl group, a 1-butynyl group, a 1-pentynyl group, a 1-hexynyl group, a 1-heptynyl group, a 1-octynyl group and a 1-undecynyl group.

Preferable examples of the substituent for the optionally substituted aromatic hydrocarbon group having 6 to 20 carbon atoms represented by Ar³, and the substituent for the optionally substituted aliphatic hydrocarbon group having 1 to 20 carbon atoms represented by Ar³ include at least one group selected from Group P2 below.

### <Group P2>

Alkoxy groups having 1 to 12 carbon atoms, alkenyloxy groups having 3 to 12 carbon atoms, alkynyloxy groups having 3 to 12 carbon atoms, and aromatic groups having 6 to 12 carbon atoms.

In Group P2, examples of the alkoxy groups having 1 to 12 carbon atoms include straight-chain or branched alkoxy groups such as a methoxy group, an ethoxy group, a propyloxy group, an isopropyloxy group, a butoxy group, an isobutoxy group, a t-butoxy group, a pentyloxy group, a hexyloxy group, a heptyloxy group, an octyloxy group, a nonyloxy group, a decyloxy group, an undecyloxy group and a dodecyloxy group; and cyclic alkoxy groups such as a cyclopropyloxy group, a cyclobutyloxy group, a cyclopentyloxy group, a cyclohexyloxy group, a cycloheptyloxy group and a cyclooctyloxy group. Examples of the alkenyloxy groups having 3 to 12 carbon atoms include a 2-propenyloxy group, a 2-butenyloxy group, a 2-methyl-2-butenyloxy group and a 3-methyl-2-butenyloxy group; examples of the alkynyloxy groups having 3 to 12 carbon atoms include a 2-propynyloxy group and a 2-butynyloxy group; and examples of the aromatic groups having 6 to 12 carbon atoms include a phenyl group, a naphthyl group, a benzofuranyl group, a benzothiophenyl group, a benzopyrazolyl group, a benzisoxazolyl group, a benzisothiazolyl group, a benzimidazolyl group, a benzoxazolyl group, a benzothiazolyl group, a quinolinyl group and an isoquinolinyl group. Herein, 1 to 3 hydrogen atoms on an aromatic ring in the aromatic groups may be each independently optionally substituted by, for example, a substituent selected form Group 3 below.

### <Group P3>

Saturated hydrocarbon groups having 1 to 12 carbon atoms, aromatic groups having 6 to 10 carbon atoms, a halogen atom, a nitro group, a trifluoromethyl group, a protected amino group and a protected hydroxyl group.

In Group P3, examples of the saturated hydrocarbon groups having 1 to 12 carbon atoms include a methyl group, an ethyl group, a propyl group, an isopropyl group, a butyl group, an isobutyl group, a t-butyl group, a pentyl group, a hexyl group, a heptyl group, an octyl group, a nonyl group, a decyl group, an undecyl group, a dodecyl group, a cyclopropyl group, a cyclobutyl group, a cyclopentyl group, a cyclohexyl group, a cycloheptyl group and a cyclooctyl group. Examples of the aromatic groups having 6 to 10 carbon atoms include a phenyl group, a 1-naphthyl group, a 2-naphthyl group, a 2-benzofuranyl group, a 3-benzofuranyl group, a 2-benzothiophenyl group, a 2-benzopyrazolyl group, a 3-benzisoxazolyl group, a 3-benzisothiazolyl group, a 2-benzimidazolyl group, a 2-benzoxazolyl group, a 2-benzothiazolyl group, a 2-quinolinyl group and a 1-isoquinolinyl group. Examples of the halogen atom include a fluorine atom, a chlorine atom and a bromine atom. Examples of the protected amino group include a benzylamino group, a 2-methoxybenzylamino group, a 2,4-dimethoxybenzylamino group, an acetylamino group, a benzyloxycarbonylamino group, a t-butoxycarbonylamino group and an allyloxycarbonylamino group. Examples of the protected hydroxyl group include straight-chain or branched alkoxy groups having 1 to 12 carbon atoms such as a methoxy group, an ethoxy group, a propoxy group, an isopropoxy group, a butoxy group, an isobutoxy group, a t-butoxy group, a pentyloxy group, a hexyloxy group, a heptyloxy group, an octyloxy group, a nonyloxy group, a decyloxy group, an undecyloxy group and a dodecyloxy group; cyclic alkyloxy groups having 3 to 12 carbon atoms such as a cyclopropyloxy group, a cyclobutyloxy group, a cyclopentyloxy group, a cyclohexyloxy group, a cycloheptyloxy group and a cyclooctyloxy group; a methoxy methoxy group; a benzyloxy group; and an acetyloxy group.

Ar³ is preferably a 1-naphthyl group, a phenyl group, a cyclohexyl group, a t-butyl group, a 1-methoxy-1,1-di-p-tolylmethyl group, a 1-methoxy-1-ethylpropyl group, a 1-methoxy-1-butylpentyl group, a 1-methoxy-1-hexylheptyl group, a 1-methoxy-1-octylnonyl group or a 3-phenyl-1-methoxy-1-(2-phenylethyl)propyl group; and more preferably, a 1-methoxy-1,1-di-p-tolylmethyl group, a 1-methoxy-1-ethylpropyl group, a 1-methoxy-1-butylpentyl group, a 1-methoxy-1-hexylheptyl group, a 1-methoxy-1-octylnonyl group or a 3-phenyl-1-methoxy-1-(2-phenylethyl)propyl group.

Examples of the optionally substituted aliphatic hydrocarbon group having 1 to 12 carbon atoms represented by R² include straight-chain or branched alkyl groups having 1 to 12 carbon atoms such as a methyl group, an ethyl group, a propyl group, an isopropyl group, a butyl group, an isobutyl group, a t-butyl group, a pentyl group, a hexyl group, a heptyl group, an octyl group, a nonyl group, a decyl group, an undecyl group and a dodecyl group; cyclic alkyl groups having 3 to 12 carbon atoms such as a cyclopropyl group, a cyclobutyl group, a cyclopentyl group, a cyclohexyl group, a cycloheptyl group and a cyclooctyl group; alkenyl groups having 3 to 12 carbon atoms such as a 2-propenyl group, a 2-butenyl group, a 2-methyl-2-butenyl group, and a 3-methyl-2-butenyl group; and alkynyl groups having 3 to 12 carbon atoms such as a 2-propynyl group and a 2-butynyl group.

The position and number of substituents of the aliphatic hydrocarbon group having 1 to 12 carbon atoms are not particularly limited. The number of substituents is preferably 1 to 3. When the group has two or more substituents, the substituents may be identical or two or more different substituents. Preferable examples of such substituents include the same substituents as those selected from Group P2 listed above.

R² is preferably a straight-chain or branched alkyl group having 1 to 12 carbon atoms, more preferably a straight-chain alkyl group having 1 to 8 carbon atoms, and even more preferably a methyl group.

Examples of straight-chain aliphatic hydrocarbon groups having 1 to 12 carbon atoms represented by R³ include straight-chain alkyl groups having 1 to 12 carbon atoms such as a methyl group, an ethyl group, a propyl group, a butyl group, a pentyl group, a hexyl group, a heptyl group, an octyl group, a nonyl group, a decyl group, an undecyl group and a dodecyl group; straight-chain alkenyl groups having 2 to 12 carbon atoms such as an ethenyl group, a 1-propenyl group, a 2-propenyl group, a 1-butenyl group, a 1-pentenyl group, a 1-hexenyl group, a 1-heptenyl group, a 1-octenyl group and a 1-undecenyl group; and straight-chain alkynyl groups having 2 to 12 carbon atoms such as an ethynyl group, a 1-propynyl group, a 2-propynyl group, a 1-butynyl group, a 1-pentynyl group, a 1-hexynyl group, a 1-heptynyl group, a 1-octynyl group and a 1-undecenyl group.

R³ is preferably a straight-chainr alkyl group having 1 to 12 carbon atoms, more preferably a straight-chain alkyl group having 1 to 8 carbon atoms, and even more preferably a methyl group.

R² and R³ may together form a polymethylene group having 2 to 6 carbon atoms. Examples of such polymethylene group having 2 to 6 carbon atoms include a trimethylene group and a tetramethylene group.

Examples of the aliphatic hydrocarbon groups having 1 to 12 carbon atoms represented by R⁴, R^{4'}, R⁵, R^{5'}, R⁶ and R^{6'} include the same aliphatic hydrocarbon groups having 1 to 12 carbon atoms which are listed above as the aliphatic hydrocarbon groups having 1 to 12 carbon atoms optionally having a substituent represented by R².

Examples of the alkoxy groups having 1 to 12 carbon atoms represented by R⁴, R^{4'}, R⁵, R^{5'}, R⁶ and R^{6'} include the same alkoxy groups having 1 to 12 carbon atoms as those listed in Group P2.

R⁴ and R^{4'} are preferably each independently an alkoxy group having 1 to 12 carbon atoms, and more preferably both are methoxy groups.

R⁵ and R^{5'} are preferably each independently an aliphatic hydrocarbon group having 1 to 12 carbon atoms, more preferably each independently a straight-chain or branched alkyl group having 1 to 8 carbon atoms, and even more preferably both are t-butyl groups.

R⁶ and R^{6'} are preferably both hydrogen atoms.

Examples of the monovalent anion represented by X⁻ include a hydroxide ion; halide ions such as a chloride ion, a bromide ion and an iodide ion; alkanesulfonate ions having 1 to 6 carbon atoms such as a methanesulfonate ion, an ethanesulfonate ion, a propanesulfonate ion, a butanesulfonate ion, a pentanesulfonate ion and a hexanesulfonate ion; and a benzenesulfonate ion. The 1 to 3 hydrogen atoms included in the benzenesulfonic acid is each independently optionally substituted by an alkyl group having 1 to 6 carbon atoms such as a methyl group, an ethyl group, a propyl group, an isopropyl group, a butyl group, an isobutyl group, a t-butyl group, a pentyl group, or a hexyl group; a halogen atom such as a fluorine atom or a chlorine atom; or a nitro group.

X⁻ is preferably a halide ion and more preferably a bromide ion.

Specific examples of compound (5) include compounds represented by Formulae (5-1) to (5-7) below and enantiomers thereof.

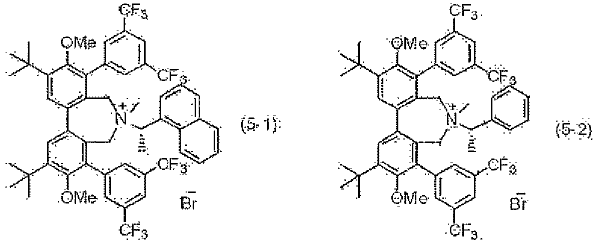

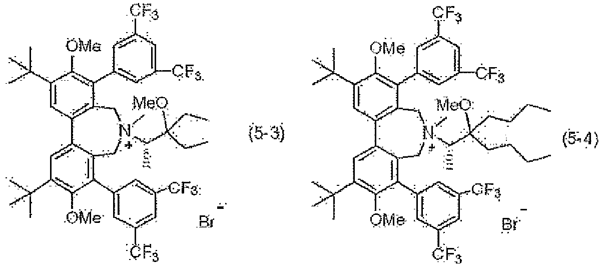

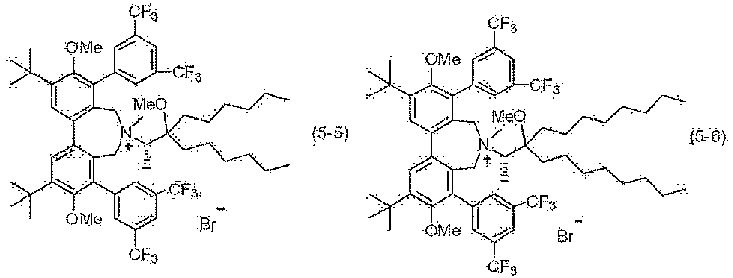

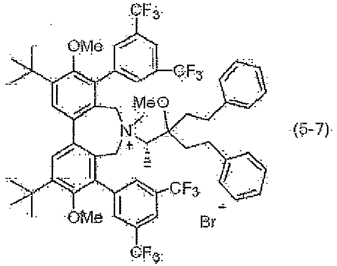

Compound (5) is prepared by reacting a compound represented by Formula (6): 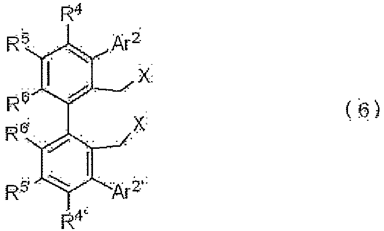 (wherein Ar², Ar^{2'}, R⁴, R^{4'}, R⁵, R^{5'}, R⁶ and R^{6'} are as defined above, and X represents a halogen atom such as a chlorine atom, a bromine atom or an iodine atom) which is prepared by the method described in Tetrahedron Letters, Volume 44, 2003, pages 5,629-5,632 with a compound represented by Formula (7): 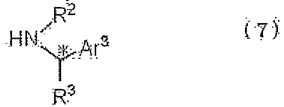 (wherein R², R³, Ar³ and * are as defined above) which is prepared from, for example, an amino acid by any of known methods, optionally in the presence of a base such as sodium hydrogen carbonate and a solvent such as acetone.

The optical purity of the optically active quaternary ammonium salt is not limited, but in order to obtain compound (3) having a high optical purity, it is preferably 90% e.e. or more, more preferably 95% e.e. or more and even more preferably 98% e.e. or more.

The reaction of compound (1) with compound (2) is preferably carried out in the presence of a base. Examples of the base used herein include alkali metal hydroxides such as sodium hydroxide, potassium hydroxide and cesium hydroxide; alkali metal carbonate compounds such as potassium carbonate and sodium carbonate; and tertiary amines such as triethylamine and diisopropylethylamine. The base is preferably an alkali metal hydroxide, and more preferably potassium hydroxide.

The reaction of compound (1) with compound (2) is preferably carried out in a solvent. Examples of the solvent include aliphatic hydrocarbon solvents, aromatic solvents, ether solvents, alcohol solvents, nitrile solvents, ester solvents, chlorinated aliphatic hydrocarbon solvents, aprotic polar solvents, and water. These solvents may be used alone, or may be used as a mixture of two or more kinds.

Examples of the aliphatic hydrocarbon solvents include pentane, hexane, isohexane, heptane, isoheptane, octane, isooctane, nonane, isononane, decane, isodecane, undecane, dodecane, cyclopentane, cyclohexane, methylcyclohexane, t-butylcyclohexane, and petroleum ether. Examples of the aromatic solvents include benzene, toluene, ethylbenzene, isopropylbenzene, t-butylbenzene, xylene, mesitylene, monochlorobenzene, monofluorobenzene, α,α,α-trifluoromethylbenzene, 1,2-dichlorobenzene, 1,3-dichlorobenzene, 1,2,3-trichlorobenzene and 1,2,4-trichlorobenzene. Examples of the ether solvents include tetrahydrofuran, methyltetrahydrofuran, diethyl ether, dipropyl ether, diisopropyl ether, dibutyl ether, dipentyl ether, dihexyl ether, diheptyl ether, dioctyl ether, t-butyl methyl ether, cyclopentyl methyl ether, 1,2-dimethoxyethane, diethylene glycol dimethyl ether, anisole and diphenyl ether. Examples of the alcohol solvent include methanol, ethanol, 1-propanol, 2-propanol, 1-butanol, isobutyl alcohol, t-butyl alcohol, 1-pentanol, 2-pentanol, isopentyl alcohol, 1-hexanol, 2-hexanol, isohexyl alcohol, 1-heptanol, 2-heptanol, 3-heptanol, isopeptyl alcohol, ethylene glycol monomethyl ether, ethylene glycol monoethyl ether, ethylene glycol monopropyl ether, ethylene glycol monoisopropyl ether, ethylene glycol monobutyl ether, ethylene glycol monoisobutyl ether, ethylene glycol mono-t-butyl ether, diethylene glycol monomethyl ether, diethylene glycol monoethyl ether, diethylene glycol monopropyl ether, diethylene glycol monoisopropyl ether, diethylene glycol monobutyl ether, diethylene glycol monoisobutyl ether, and diethylene glycol mono-t-butyl ether. Examples of the nitrile solvents include acetonitrile, propionitrile, and benzonitrile. Examples of the ester solvents include ethyl acetate, propyl acetate, isopropyl acetate, butyl acetate, isobutyl acetate, t-butyl acetate, amyl acetate, and isoamyl acetate. Examples of the chlorinated aliphatic hydrocarbon solvents include dichloromethane, chloroform, and 1,2-dichloroethane. Examples of the aprotic polar solvents include dimethyl sulfoxide, sulfolane, N,N-dimethylformamide, N,N-dimethylacetamide, N,N-dimethylpropionamide, N-methylpyrrolidone, γ-butyrolactone, dimethyl carbonate, diethyl carbonate, ethylene carbonate, propylene carbonate, 1,3-dimethyl-2-imidazolidinone, and 1,3-dimethyl-3,4,5,6-tetrahydro-2(1H)-pyridinone. The solvent is preferably used as a mixture of water and solvent(s) other than water; preferably as a mixture of water and the aromatic solvent(s) or the ether solvent(s); and more preferably as a mixture of water and toluene or t-butyl methyl ether.

In the reaction of compound (1) with compound (2), the amount of compound (2) used is preferably 0.8 to 20 mol, and more preferably 0.9 to 5 mol per 1 mol of compound (1).

In the reaction of compound (1) with compound (2), the amount of the optically active quaternary ammonium salt is not limited, but is preferably 0.00001 to 0.5 mol, and more preferably 0.001 to 0.1 mol per 1 mol of compound (1).

In the reaction of compound (1) with compound (2), the amount of the base used is preferably 2 to 30 mol, and more preferably 4 to 15 mol per 1 mol of compound (1).

When the reaction of compound (1) with compound (2) is carried out in a solvent, the amount of the solvent is not particularly limited, but the amount is preferably 1 to 100 mL, and more preferably 3 to 30 mL per 1 g of compound (1).

The reaction temperature is preferably in the range of -30 to 70°C, and more preferably in the range of -10 to 40°C. The reaction time depends on the amount of the used optically active quaternary ammonium salt, the reaction temperature and the like, but is preferably in the range of 1 to 120 hours.

The progress of the reaction can be confirmed by, for example, an analysis means such as gas chromatography or liquid chromatography.

The method for mixing the reaction reagents is not limited. Examples thereof include a method wherein compound (1) is mixed with a solvent as needed, compound (2) and an optically active quaternary ammonium salt are added thereto, then the temperature of the obtained mixture is controlled to the reaction temperature, and thereafter, a base is added to the mixture controlled to have the reaction temperature.

The optical purity of compound (3) obtained from the reaction of compound (1) with compound (2) is, for example, 40% e.e. or more and less than 95% e.e., for example, 55% e.e. or more and less than 95% e.e., for example, 70% e.e. or more and less than 90% e.e. or for example, 75% e.e. or more and less than 85% e.e. when compound (5) is used as the optically active quaternary ammonium salt.

The obtained compound (3) may be isolated, or may be used in the subsequent step without being isolated. When it is isolated, the reaction mixture after completion of the reaction is subjected to an aftertreatment such as neutralization, extraction-and-washing, washing with water, or concentration; and as needed, subjected to adsorption using activated carbon, silica, alumina, or the like, and to purification such as recrystallization, distillation or silica gel column chromatography.

Compound (4-2) is obtained by subjecting compound (3) to imine hydrolysis. As used herein, imine hydrolysis refers to a reaction of converting an arylmethylidene amino group in compound (3) into an amino group.

The imine hydrolysis is not particularly limited as far as it is a method wherein an ester site contained in compound (3) is not hydrolyzed. Preferably, the imine hydrolysis is carried out by mixing compound (3) with an acid.

Examples of the acid which is used in the imine hydrolysis include inorganic acids such as hydrochloric acid, sulfuric acid, phosphoric acid, nitric acid and perchloric acid; aromatic sulfonic acids such as p-toluenesulfonic acid and benzenesulfonic acid; aliphatic sulfonic acids such as methanesulfonic acid; aliphatic carboxylic acids such as acetic acid, propionic acid, citric acid, malic acid, succinic acid, lactic acid, maleic acid and fumaric acid; as well as aromatic carboxylic acids such as phthalic acid, benzoic acid, 4-nitrobenzoic acid and 4-chlorobenzoic acid.

The acid may be used alone, or may be used as a mixture of two or more kinds. The acid may be used as a mixture with the solvent described later.

The acid is preferably an inorganic acid, and more preferably hydrochloric acid. The concentration of hydrochloric acid may be appropriately controlled when it is used.

The amount of the acid used in the imine hydrolysis is preferably controlled so that the mixture obtained after mixing the acid has a pH in the range of 0 to 4. In order to control the pH within this range, for example, 0.8 to 1.5 mol of hydrochloric acid may be used per 1 mol of compound (3) when the acid is hydrochloric acid.

The imine hydrolysis is preferably carried out in a solvent. The solvent used in the imine hydrolysis may be, for example, the same solvent as that used in the reaction of compound (1) with compound (2), and is preferably water, an aromatic solvent or an ether solvent.

The amount of the used solvent is preferably 1 to 100 mL, and more preferably 3 to 30 mL per 1 g of compound (3).

The temperature for conducting the imine hydrolysis is generally in the range of 0 to 80°C, preferably in the range of 5 to 60°C, and more preferably in the range of 10 to 40°C.

The time for conducting the imine hydrolysis is preferably in the range of 1 minute to 20 hours, and more preferably in the range of 10 minute to 10 hours, although it depends on the kind and the concentration of the acid used, or on the temperature at the time of the imine hydrolysis.

The method for mixing the ingredients in the imine hydrolysis is not limited, but examples thereof include a method wherein compound (3) and a solvent are mixed with each other, and then an acid is added to the obtained mixture.

The optical purity of compound (4-2) obtained by the imine hydrolysis is nearly equal to the optical purity of compound (3) which is subjected to the imine hydrolysis. Specifically, when compound (5) is used as the optically active quaternary ammonium salt in the reaction of compound (1) with compound (2), the optical purity of the obtained compound (4-2) is, for example, 40% e.e. or more and less than 95% e.e., for example, 55% e.e. or more and less than 95% e.e., for example, 70% e.e. or more and less than 90% e.e., or for example, 75% e. e. or more and less than 85% e.e.

The obtained compound (4-2) may be isolated, or may be used in the production method of the present invention without being isolated. It is also possible to subject the reaction mixture obtained from the imine hydrolysis to the production method of the present invention after subjecting the mixture to aftertreatments, for example, neutralization, extraction-and-washing, washing with water, and concentration.

### EXAMPLES

Hereinafter, the present invention is explained further in detail with reference to examples.

### <Production Example 1> (Production of ((E)-N-phenylmethylene glycine ethyl ester)

Glycine ethyl ester hydrochloride (13.8 g, 98.9 mmol) was mixed with 50 g of toluene, and 10g of dimethyl sulfoxide was added to the obtained mixture at room temperature, and 10.0 g (94.2 mmol) of benzaldehyde was further added thereto. The obtained mixture was stirred at a temperature of 12°C, and then 16.5 g of a 25% by weight aqueous sodium hydroxide solution (sodium hydroxide 104 mmol) was added dropwise over 3 hours. After the dropwise addition was completed, the obtained mixture was stirred at a temperature of 11°C to 13°C for 20 hours. After the reaction was completed, the reaction mixture was cooled to 5°C, and 11.4 mL of water was added dropwise. Then the stirring was stopped, and the solution was subjected to separation to obtain an organic layer. The organic layer was then washed with 19 g of a 20% by weight saline solution. After the organic layer was dried over magnesium sulfate, the solvent was distilled off under reduced pressure to thereby obtain 43.6 g of a toluene solution of (E)-N-phenylmethyleneglycine ethyl ester ((E)-N-phenylmethyleneglycine ethyl ester content 16.5 g) (yield 92%).

### <Production Example 2> (Production of ethyl

### 1-amino-2-vinylcyclopropanecarboxylate (abbreviated as ethyl ester A)

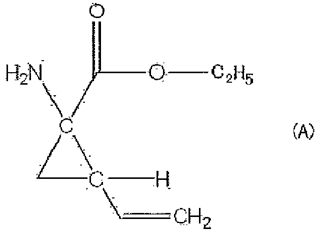

The toluene solution (2.60 g) of (E)-N-phenylmethyleneglycine ethyl ester obtained in Production Example 1 ((E)-N-phenylmethyleneglycine ethyl ester content 0.98 g, 5.14 mmol) was mixed with 10 mL of toluene. To the obtained mixture were added 1.00 g (4.68 mol) of (E)-1,4-dibromo-2-butene and 0. 028 g (0.023 mmol) of a compound represented by Formula (5-6) at room temperature. The obtained mixture was cooled to 0°C, and 5.25 g of a 50% aqueous potassium hydroxide solution (potassium hydroxide 46.8 mmol) was added dropwise thereto and stirred at a temperature of 0°C for 20 hours. After the reaction was completed, to the obtained mixture was added 3 mL of water. Then the stirring was stopped, and the solution was subj ected to liquid separation to obtain an organic layer. The organic layer was then washed with 3 mL of a 20% by weight saline solution. After the liquid separation was carried out, an organic layer of the mixture was obtained, which contained ethyl (1R,2S)-1-(N-phenylmethylene)amino-2-vinylcyclopropanecarboxylate in an amount which is larger than that of an enantiomer thereof.

Subsequently, 4.7 mL of 1 M aqueous hydrochloric acid solution was added to the obtained organic layer, and then stirred for 2 hours at room temperature to perform imine hydrolysis. After the reaction was completed, 3 mL of water was added to an organic layer obtained by the liquid separation and extracted. The thus obtained aqueous layer was combined, and 7.93 g of an aqueous solution of ethyl ester A was obtained, which contained (1R,2S)-1-amino-2-vinylcyclopropanecarboxylic acid ethyl ester hydrochloride in an amount which is larger than that of an enantiomer thereof. The resultant aqueous solution was analyzed under the conditions for high performance liquid chromatography analysis and the conditions for optical purity analysis below, to calculate the yield and the optical purity of the ethyl ester A. Yield 66%. Optical purity 79% e.e.

### <Conditions for High Performance Liquid Chromatography Analysis>

Column: YMC Pack ODS-A-302 (4.6 × 150 mm, 5 µm)
Mobile phase: A = 40 mM KH₂PO₄ Water (pH 3.5 - H₃PO₄),
   B = methanol
   A/B = 10% (0 min) → 10% (5 min) → 70% (25 min)
→ 70% (45 min)
Flow rate: 1.0 mL/minute
Detector: Wavelength 220 nm
Retention time: 11.7 minutes (ethyl (1R,2S)-1-(N-phenylmethylene)amino-2-vinylcyclopropanecarboxylate)
<Conditions for Optical Purity Analysis>
Column: CHIRALPAK (Daicel Chemical Industries, registered trademark) AD-RH (4.6 × 150 mm, 5 µm)
Mobile phase: A = 20 mM aqueous solution of dipotassium hydrogenphosphate (prepared to be pH 8.0 with phosphoric acid),
   B = acetonitrile
   A/B = 80/20
Flow rate: 0.5 mL/minute
Detector: wavelength 215 nm
Retention time: (1R,2S) isomer = 14.7 minutes, (1S,2R) isomer = 16.2 minutes

### <Example 1>

To 8.17 g of the aqueous solution of ethyl ester A obtained in Production Example 2 (optical purity 79% e. e. , 3.1 mmol) was added 20 mL of toluene at room temperature. To the obtained mixture was further added dropwise 0.39 g of a 48% by weight aqueous sodium hydroxide solution. After the dropwise addition and subsequent 10 minutes of stirring, the stirring was stopped and liquid separation was carried out to separate the organic layer from the solution. To the obtained organic layer was added 10 mL of 2-propanol and stirred. Then, 0.46 g (3.1 mmol) of L-tartaric acid was added thereto. The obtained mixture was stirred overnight at room temperature, and the resultant slurry was filtered to obtain crystals. The thus obtained crystals were washed with a mixed solvent of 2 mL of toluene and 0.5 mL of 2-propanol, and then dried under reduced pressure to thereby obtain 0.81 g (2.6 mmol) of an L-tartrate of ethyl (1R,2S)-1-amino-2-vinylcyclopropanecarboxylate (yield 83%). The resultant crystals were analyzed under the conditions for optical purity analysis described in Production Example 2 in order to determine the optical purity thereof. Optical purity 90% e.e.

### Ethyl (1R,2S)-1-amino-2-vinylcyclopropanecarboxylate L-tartrate

¹H-NMR (CD₃OD, 400 MHz) δppm: 5.78-5.67 (1H, m), 5.35 (1H, dd, J = 1.4, 17.1 Hz), 5.16 (1H, dd, J = 1.4, 10.3 Hz), 4.42 (2H, s), 4.30-4.22 (2H, m), 2.34 (1H, q, J = 8.8 Hz), 1.73-1.64 (2H, m), 1.30 (3H, t, J = 6.8 Hz).

The obtained ethyl (1R,2S)-1-amino-2-vinylcyclopropanecarboxylate L-tartrate is mixed with toluene and water, and a 25% by weight aqueous sodium hydroxide solution is added dropwise to the resultant mixture. After the resultant mixture is stirred, an organic layer is separated therefrom to obtain a toluene solution which contains ethyl
(1R,2S)-1-amino-2-vinylcyclopropanecarboxylate. A salt of ethyl (1R,2S)-1-amino-2-vinylcyclopropanecarboxylate can be obtained by adding an acid such as sulfuric acid to the obtained toluene solution of ethyl
(1R,25S)-1-amino-2-vinylcyclopropanecarboxylate.

### <Production Example 3> (Production of ethyl ester A)

The toluene solution (2.60 g) of (E)-N-phenylmethyleneglycine ethyl ester obtained in Production Example 1 ((E)-N-phenylmethyleneglycine ethyl ester content 0.98 g, 5.14 mmol) was mixed with 10 mL of toluene. To the obtained mixture were added 1.00 g (4.68 mmol) of (E)-1,4-dibromo-2-butene and 0.027 g (0.023 mmol) of a compound represented by Formula (5-7) at room temperature. The obtained mixture was cooled to 0°C, and 5.25 g of a 50% by weight aqueous potassium hydroxide solution (potassium hydroxide 46.8 mmol) was added dropwise thereto and stirred at a temperature of 0°C for 20 hours. After the reaction was completed, to the obtained mixture was added 3 mL of water, and the mixture was subjected to liquid separation. The resultant organic layer was washed with 3 mL of a 20% by weight saline solution to thereby obtain an organic layer which contained ethyl (1R,2S)-1-(N-phenylmethylene)amino-2-vinylcyclopropanecarboxylate in an amount which is larger than that of an enantiomer thereof.

Subsequently, 4.7 mL of 1 M hydrochloric acid was added to the obtained organic layer, then stirred for 2 hours at room temperature to perform imine hydrolysis. After the reaction was completed, to the separated organic layer was added 3 mL of water and extracted. The thus obtained aqueous layer was combined, and 7.93 g of an aqueous solution of ethyl ester A was obtained, which contained (1R,2S)-1-amino-2-vinylcyclopropanecarboxylic acid ethyl ester hydrochloride in an amount which is larger than that of an enantiomer thereof. The resultant aqueous solution was analyzed under the conditions for high performance liquid chromatography analysis and optical purity analysis described in Production Example 2 in order to calculate the yield and the optical purity of ethyl ester A. Yield 67%. Optical purity 84% e.e.

### <Example 2>

To 8.25 g of the aqueous solution of ethyl ester A obtained in Production Example 3 (optical purity 84% e.e., 3.1 mmol) was added 20 mL of toluene at room temperature. To the obtained mixture was further added dropwise 0.39 g of a 48% by weight aqueous sodium hydroxide solution. After the dropwise addition, the mixture was stirred for 10 minutes, and subjected to liquid separation to thereby separate an organic layer from the mixture. To the organic layer was added 10 mL of 2-propanol. To the thus obtained mixture was added 0.73 g (3.1 mmol) of D-10-camphorsulfonic acid, and stirred overnight at room temperature. The resultant mixture was concentrated under reduced pressure so that 2-propanol was distilled off to thereby precipitate crystals. The thus obtained slurry was stirred for 2 hours and then filtered. The thus obtained crystals were washed with 2 mL of toluene, and then dried under reduced pressure to thereby obtain 0.95 g (2.5 mmol) of ethyl (1R,2S)-1-amino-2-vinylcyclopropanecarboxylate D-10-camphorsulfonate (yield 78%). The resultant crystals were analyzed under the conditions for optical purity analysis described in Production Example 2 in order to determine the optical purity thereof. Optical purity 99% e.e.

### Ethyl (1R,2S)-1-amino-2-vinylcyclopropanecarboxylate D-10-camphorsulfonate

¹H-NMR (CD₃OD, 400 MHz) δppm: 5.79-5.69 (1H, m), 5.40 (1H, dd, J = 1.4, 17.1 Hz), 5.23 (1H, dd, J = 1.4, 10.3 Hz), 4.35-4.23 (2H, m), 2.76 (1H, d, J = 15.1 Hz), 2.70-2.60 (1H, m), 2.43-2.28 (2H, m), 2.08-1.96 (2H, m), 1.89 (1H, d, J = 18.6 Hz), 1.82-1.68 (2H, m), 1.65-1.56 (1H, m), 1.45-1.36 (1H, m), 1.31 (3H, t, J = 6.8 Hz), 1.12 (3H, s), 0.85 (3H, s).

Ethyl (1R,2S)-1-amino-2-vinylcyclopropanecarboxylate D-10-camphorsulfonate is mixed with toluene and water, and a 25% by weight aqueous sodium hydroxide solution is added dropwise to the resultant mixture. After the resultant mixture is stirred, an organic layer is separated therefrom to obtain a toluene solution which contains ethyl
(1R,2S)-1-amino-2-vinylcyclopropanecarboxylate. A salt of ethyl (1R,2S)-1-amino-2-vinylcyclopropanecarboxylate can be obtained by adding an acid such as sulfuric acid to the obtained toluene solution of ethyl
(1R,2S)-1-amino-2-vinylcyclopropanecarboxylate.

### <Production Example 4> (Production of ethyl ester A)

The toluene solution (75.0 g) of (E)-N-phenylmethyleneglycine ethyl ester obtained in Production Example 1 ((E)-N-phenylmethyleneglycine ethyl ester content: 18.8 g, 98.2 mmol) was mixed with 84 g of toluene. To the obtained mixture were added 20.0 g (93.5 mmol) of (E)-1,4-dibromo-2 -but ene and 1.31 g (9.4 mmol) of glycine ethyl ester hydrochloride and 0.29 g (0.28 mmol) of a compound represented by Formula (5-1) at room temperature. The obtained mixture was cooled to 0°C, and 42.0 g of a 50% by weight aqueous potassium hydroxide solution (potassium hydroxide 748 mmol) was added dropwise thereto over 3 hours and stirred at a temperature of 0°C for 16 hours. After the reaction was completed, to the obtained mixture was added 60 mL of water. Then the stirring was stopped, and the solution was subjected to liquid separation. The resultant organic layer was washed with 60 g of a 20% by weight saline solution. After liquid separation was carried out, an organic layer was obtained, which contained ethyl (1R,2S)-1-(N- phenylmethylene)amino-2-vinylcyclopropanecarboxylate in an amount which is larger than that of an enantiomer thereof.

Subsequently, 27.0 g of water was added to the obtained organic layer at room temperature, and 8.77 g of 35% by weight hydrochloric acid was added dropwise at room temperature over 20 minutes, followed by imine hydrolysis at room temperature for 2 hours. After the imine hydrolysis was completed, the aqueous layer was separated out, and to the organic layer was added 18.3 g of 0.5% by weight hydrochloric acid at room temperature and extracted. The thus obtained aqueous layer was combined, and 66.6 g of an aqueous solution of ethyl ester A was obtained, which contained (1R,2S)-1-amino-2-vinylcyclopropanecarboxylic acid ethyl ester hydrochloride in an amount which is larger than that of an enantiomer thereof. The resultant aqueous solution was analyzed under the conditions for high performance liquid chromatography analysis and optical purity analysis described in Production Example 2 in order to calculate the yield and the optical purity of the ethyl ester A. Yield 65%. Optical purity 78% e.e.

### <Example 3>

From the aqueous solution of ethyl ester A obtained in Production Example 4 (optical purity 78% e.e., 9.05 mmol), 10.0 g of the same was separated. Into the separated solution, 12 g of toluene was poured at room temperature. To the obtained mixture was added dropwise 1.11 g of a 48% by weight aqueous sodium hydroxide solution. After the dropwise addition and subsequent 20 minutes of stirring, the stirring was stopped and an organic layer was separated from the solution. To the obtained aqueous layer was added 3. 0 g of toluene and the mixture was subjected to extraction. The thus obtained organic layer together with the previously separated organic layer were dried over magnesium sulfate, and then 4.5 g of ethanol, and further 1.36 g (9.05 mmol) of L-tartaric acid were added thereto. The thus obtained mixture was stirred overnight at room temperature, and then cooled in an ice bath with stirring for 5 hours. The resultant slurry was filtered to separate crystals therefrom. The separated crystals were washed with a mixed solvent of 3.0 g of toluene and 0.3 g of ethanol. The washed crystals were dried under reduced pressure to thereby obtain 2.09 g (6.85 mmol) of ethyl (1R,2S)-1-amino-2-vinylcyclopropanecarboxylate L-tartrate (yield 76%). The resultant crystals were analyzed under the conditions for optical purity analysis described in Production Example 2 in order to determine the optical purity thereof. Optical purity 97% e.e.

### <Production Example 5> (Production of ethyl ester A)

The toluene solution (141.6 g) of (E)-N-phenylmethyleneglycine ethyl ester obtained in Production Example 1 ((E)-N-phenylmethyleneglycine ethyl ester content: 33.8 g, 177 mmol) was mixed with 180 g of toluene. To the obtained mixture were added 36.0 g (168 mmol) of (E)-1,4-dibromo-2-butene and 0. 53 g (0.51 mmol) of a compound represented by Formula (5-1) at room temperature. The obtained mixture was cooled to 0°C, and 151 g of a 50% by weight aqueous potassium hydroxide solution (potassium hydroxide 1,346 mmol) was added dropwise thereto over 3 hours and stirred at a temperature of 0°C for 24 hours. After the reaction was completed, to the obtained mixture was added 108 g of water. Then the stirring was stopped, and the solution was subjected to separation. The resultant organic layer was washed with 108 g of a 20% by weight saline solution. After the liquid separation, an organic layer was obtained, which contained ethyl (1R,2S)-1-(N-phenylmethylene)amino-2-vinylcyclopropanecarboxylate in an amount which is larger than that of an enantiomer thereof.

Subsequently, 48.6 g of water was added to the obtained organic layer at room temperature, and 15.8 g of 35% by weight hydrochloric acid was added dropwise at room temperature over 20 minutes, followed by imine hydrolysis at room temperature for 2 hours. After the imine hydrolysis was completed, the aqueous layer was separated out, and to the organic layer was added 32.4 g of a 0.5% by weight aqueous hydrochloric acid solution at room temperature and extraction was carried out. The thus obtained aqueous layer was combined, and 118.5 g of an aqueous solution of ethyl ester A was obtained, which contained (1R,2S)-1-amino-2-vinylcyclopropanecarboxylicacid ethyl ester hydrochloride in an amount which is larger than that of an enantiomer thereof. The resultant aqueous solution was analyzed under the conditions for high performance liquid chromatography analysis and optical purity analysis described in Production Example 2, to calculate the yield and the optical purity of ethyl ester A. Yield 64%. Optical purity 76% e.e.

### <Example 4>

From the aqueous solution of ethyl ester A obtained in Production Example 5 (optical purity 76% e.e., 14.9 mmol), 15.6 g of the same was separated. Into the separated aqueous solution, 15 g of isopropyl acetate was added at room temperature and further 1.85 g of a 48% by weight aqueous sodium hydroxide solution was added dropwise. After the dropwise addition, the mixture was stirred for 20 minutes and an organic layer was separated from the mixture. To the resultant aqueous layer was added 5.0 g of isopropyl acetate and extraction was carried out. An organic layer obtained by the extraction together with the previously obtained organic layer by the separation were dried over magnesium sulfate to thereby obtain an isopropyl acetate solution of ethyl ester A.

From the obtained solution, 5.97 g of the same was separated (ethyl ester A, 2.96 mmol), and then 0.44 g (2.6 mmol) of L-tartaric acid was added and stirred. To the thus obtained mixture was added 2 mL of ethanol, and the mixture was stirred overnight at room temperature and then cooled in an ice bath with stirring for 2 hours. A supernatant of the obtained slurry was analyzed under the conditions for optical purity analysis described in Production Example 2. The optical purity thereof was 19% e.e. By filtering the obtained slurry, it is possible to obtain ethyl (1R,2S)-1-amino-2-vinylcyclopropanecarboxylate L-tartrate having an optical purity of 90% e.e. or more.

### <Example 5>

Ethyl ester A (optical purity 76% e.e., 17.8 mmol) was separated from the same obtained in Production Example 5. To the separated aqueous solution was added 18 g of diisopropyl ether at room temperature and stirred, followed by further dropwise addition of 2.22 g of a 48% by weight aqueous sodium hydroxide solution. After the dropwise addition, the mixture was stirred for 20 minutes and the organic layer was separated from the mixture. To the resultant aqueous layer was added 6.0 g of diisopropyl ether and extraction was carried out. The organic layer obtained by the extraction together with the previously obtained organic layer by the separation were dried over magnesium sulfate to thereby obtain a diisopropyl ether solution of ethyl ester A.

From the obtained solution, 9.46 g of the same was separated (ethyl ester A, 5.66 mmol), which was then added dropwise to a mixture of 2.0 g of ethanol, 2.0 g of 2-propanol and 0.85 g (5.66 mmol) of L-tartaric acid at room temperature. The resultant mixture was stirred for 1 hour at room temperature, and then cooled in an ice bath with stirring for 2 hours. A supernatant of the obtained slurry was analyzed under the conditions for optical purity analysis described in Production Example 2. The optical purity thereof was 41% e.e. By filtering the obtained slurry, it is possible to obtain crystals having an optical purity of 90% e.e. or more.

### <Example 6>

From the aqueous solution of ethyl ester A obtained in Production Example 5, 28.78 g of the same was separated, and then 26.2 g of toluene was added at room temperature. To the obtained mixture was added dropwise 3.24 g of a 48% by weight aqueous sodium hydroxide solution. After the dropwise addition and subsequent 30 minutes of stirring, the stirring was stopped and an organic layer was separated from the solution. To the resultant aqueous layer was added 8.8 g of toluene and extraction was carried out. An organic layer obtained by the extraction together with the previously obtained organic layer by the separation were washed with 8.8 g of a 20% by weight saline solution, and then dried over magnesium sulfate to thereby obtain a toluene solution of ethyl ester A.

From the obtained toluene solution, 34.5 g of the same was separated (ethyl ester A, 20. 3 mmol), which was then added dropwise to a mixture of 10.5 g of ethanol and 3.50 g (23.3 mmol) of L-tartaric acid at a temperature of 30°C. The resultant mixture was stirred for 15 hours at room temperature. The resultant slurry was filtered to separate crystals therefrom. The obtained crystals were washed with a mixed solvent of 7.0 g of toluene and 2.1 g of ethanol and dried under reduced pressure to thereby obtain 5.49 g of crystals. The thus obtained crystals were analyzed under the conditions for the quantitative analysis described in Production Example 2 to thereby determine the content of ethyl (1R,2S)-1-amino-2-vinylcyclopropanecarboxylate L-tartrate. The content of ethyl (1R,2,S)-1-amino-2-vinylcyclopropanecarboxylate L-tartrate was 5.00 g (16.4 mmol) (yield 81%). The resultant crystals were analyzed under the conditions for optical purity analysis described in Production Example 2 in order to determine the optical purity thereof. Optical purity 94% e.e.

From the obtained crystals, 1.50 g (ethyl (1R,2S)-1-amino-2-vinylcyclopropanecarboxylate L-tartrate, content 1.37 g (4.48 mmol)) was separated, and then 6.0 g of ethanol and 6.0 g of methanol were added and stirred. The resultant mixture was heated in a 40°C bath to dissolve the crystals. While heated in the 40°C bath, the solution was concentrated under reduced pressure so that 7 g of the solvent was distilled off. The obtained concentrate was inoculated with ethyl (1R,2S)-1-amino-2-vinylcyclopropanecarboxylate L-tartrate (optical purity 100% e.e.). The thus obtained slurry was cooled down to room temperature, and then 6.0 g of toluene was added dropwise. After 2 hours of stirring at room temperature, the slurry was cooled in an ice bath with stirring for 2 hours. The resultant slurry was filtered to separate crystals therefrom. The obtained crystals were washed with a mixed solvent of 1.5 g of toluene and 0.75 g of ethanol, and then dried under reduced pressure to thereby obtain 1.08 g (3.54 mmol) of ethyl (1R,2S)-1-amino-2-vinylcyclopropanecarboxylate L-tartrate. Yield 79%. The resultant crystals were analyzed under the conditions for optical purity analysis described in Production Example 2 in order to determine the optical purity thereof. Optical purity 100% e.e.

### INDUSTRIAL APPLICABILITY

Optically active 1-amino-2-vinylcyclopropanecarboxylic acid esters are useful, for example, as a synthetic intermediate of pharmaceuticals such as an antiviral agent.

The present invention is useful because it provides a method of producing an optically active 1-amino-2-vinylcyclopropanecarboxylic acid ester.

## Claims

1. A method of producing an optically active 1-amino-2-vinylcyclopropanecarboxylic acid ester, comprising:
reacting a 1-amino-2-vinylcyclopropanecarboxylic acid ester with optically active tartaric acid or optically active camphorsulfonic acid in a solvent to thereby obtain a mixture of diastereomeric salts and isolating one of the diastereomeric salts from the thus obtained mixture; and
treating the isolated diastereomeric salt with an inorganic acid or a base.

2. The method of claim 1, wherein the 1-amino-2-vinylcyclopropanecarboxylic acid ester is a compound represented by Formula (4-2): 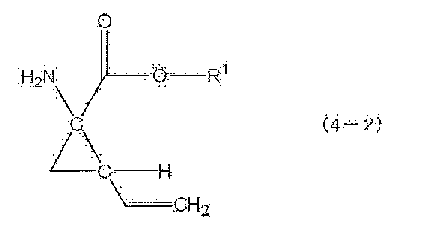 (wherein R¹ represents an alkyl group having 1 to 12 carbon atoms or an alkenyl group having 2 to 12 carbon atoms), and the resultant optically active 1-amino-2-vinylcyclopropanecarboxylic acid ester is a compound represented by Formula (4): 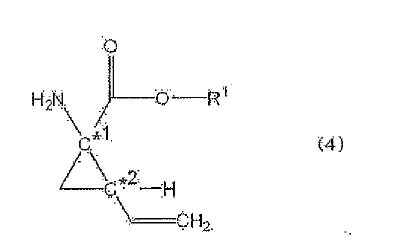 (wherein R¹ is as defined above; C^{*1} and C^{*2} each represent an asymmetric carbon atom; C*² has an S configuration when C*¹ has an R configuration, and C*² has an R configuration when C*¹ has an S configuration).

3. The method of claim 1, wherein the reaction of the 1-amino-2-vinylcyclopropanecarboxylic acid ester with the optically active tartaric acid or the optically active camphorsulfonic acid is carried out in an aromatic solvent, a ketone solvent, an ester solvent, an alcohol solvent, an ether solvent, or a mixture thereof.

4. The method of claim 1, wherein the reaction of the 1-amino-2-vinylcyclopropanecarboxylic acid ester with the optically active tartaric acid or the optically active camphorsulfonic acid is carried out in a mixed solvent of an aromatic solvent and an alcohol solvent.

5. A method of producing a compound represented by Formula (4), comprising:
reacting a compound represented by Formula (1): 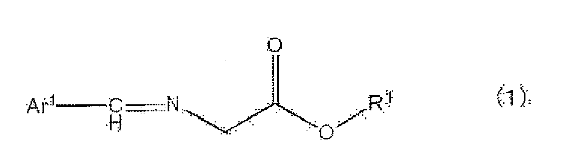 (wherein R¹ represents an alkyl group having 1 to 12 carbon atoms or an alkenyl group having 2 to 12 carbon atoms, Ar¹ represents an optionally substituted phenyl group or an optionally substituted naphthyl group) with a compound represented by Formula (2): 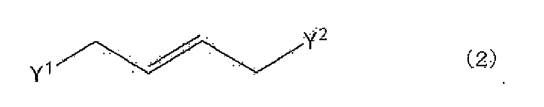 (wherein Y¹ and Y² represent each independently a halogen atom, an alkanesulfonyloxy group having 1 to 6 carbon atoms, a perfluoroalkanesulfonyloxy group having 1 to 6 carbon atoms or a benzenesulfonyloxy group; wherein the hydrogen atoms contained in the benzenesulfonyloxy group is each independently optionally substituted by a group selected from the group consisting of an alkyl group having 1 to 6 carbon atoms, a halogen atom and a nitro group) in the presence of an optically active quaternary ammonium salt, to thereby obtain a compound represented by Formula (3): 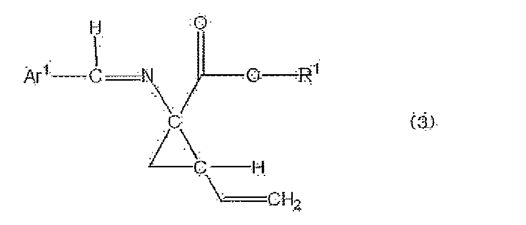 (wherein Ar¹ and R¹ are as defined above);
subjecting the thus obtained compound represented by Formula (3) to imine hydrolysis, to thereby obtain a compound represented by Formula (4-2): 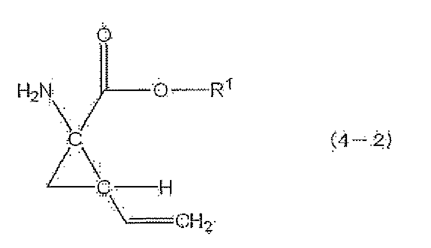 (wherein R¹ is as defined above);
reacting the obtained compound represented by Formula (4-2) with optically active tartaric acid or optically active camphorsulfonic acid in a solvent, to thereby obtain a mixture of diastereomeric salts;
isolating one of the diastereomeric salts from the thus obtained mixture; and
treating the isolated diastereomeric salt with an inorganic acid or a base, to thereby obtain the compound represented by Formula (4): 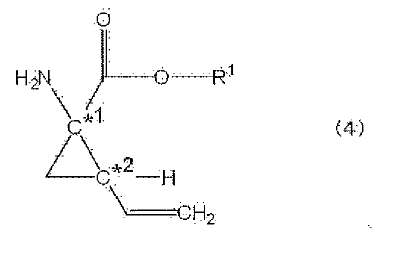 (wherein R¹ is as defined above; C^{*1} and C^{*2} each represent an asymmetric carbon atom; and C*² has an S configuration when C*¹ has an R configuration, and C*² has an R configuration when C*¹ has an S configuration).

6. The method of claim 5, wherein the optically active quaternary ammonium salt is an optically active compound represented by Formula (5): 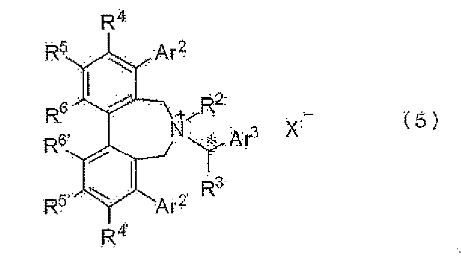 (wherein Ar² and Ar^{2'} represent each independently an optionally substituted phenyl group; Ar³ represents an optionally substituted aromatic hydrocarbon group having 6 to 20 carbon atoms or an optionally substituted aliphatic hydrocarbon group having 1 to 20 carbon atoms; R² represents an optionally substituted aliphatic hydrocarbon group having 1 to 12 carbon atoms and R³ represents a straight-chain hydrocarbon group having 1 to 12 carbon atoms, or alternatively, R² and R³ in combination form a polymethylene group having 2 to 6 carbon atoms; R⁴, R^{4'}, R⁵, R^{5'}, R⁶ and R^{6'} represent each independently a hydrogen atom, an aliphatic hydrocarbon group having 1 to 12 carbon atoms or an alkoxy group having 1 to 12 carbon atoms; * represents an asymmetric carbon atom; and X⁻ represents a monovalent anion).

7. The method of claim 6, wherein Ar² and Ar^{2'} are both 3,5-bis(trifluoromethyl)phenyl groups, R⁶ and R^{6'} are both hydrogen atoms, and R² and R³ are each independently an alkyl group having 1 to 12 carbon atoms.

8. The method of claim 6, wherein Ar³ is an aromatic hydrocarbon group having 6 to 20 carbon atoms, which has an alkoxy group having 1 to 12 carbon atoms; or an aliphatic hydrocarbon group having 1 to 20 carbon atoms, which has an alkoxy group having 1 to 12 carbon atoms.

9. A salt of a (1R,2S)-1-amino-2-vinylcyclopropanecarboxylic acid ester or a (1S,2R)-1-amino-2-vinylcyclopropanecarboxylic acid ester and optically active tartaric acid or optically active camphorsulfonic acid.
